# EUROPEAN PATENT APPLICATION

(11) **EP 1 308 511 A1**
(43) Date of publication of application: **07.05.2003**
(21) Application number: 02021286.6
(22) Date of filing: 19.09.2002
(51) Int. Cl.: C12N 15/11, C12N 15/12, C12N 5/10, A01K 67/027, C07K 14/515, C12N 15/63, G01N 33/50, A61K 38/17, A61P 7/06, A61P 35/02

(54) **Use of angiopoietin-like 1 and angiopoietin-like 2 nucleic acids and proteins for the treatment of blood-related disorders and defects in vasculature.**

(30) Priority: 31.10.2001 US 335362 P
(71) Applicant: Mermaid Pharmaceuticals GmbH, 20251 Hamburg (DE)
(72) Inventor: Esguerra, Camila V., 25337 Seeth-Ekholt (DE)
(74) Representative: Grund, Martin, Dr.

(57) **Abstract**

The present invention relates to methods of using ANGPTL1 and ANGPTL2 genes for altering clinical status. In particular, the present invention provides pharmaceutical compositions and antisense oligomers comprising polynucleotide sequences and amino acids sequences of the angiopoietin-like protein genes, ANGPTL1 and ANGPTL2. Furthermore, methods for screening for therapeutic agents, methods for determining whether a subject is at risk for a condition wherein one or more cellular processes of erythroid growth, differentiation, maturation and survival is altered using polynucleotide sequences and amino acids sequences of the angiopoietin-like protein genes, ANGPTL1 and ANGPTL2. Moreover, the invention provides transgenic non-human animals lacking functional ANGPTL1 or ANGPTL2.

## Description

### ABSTRACT

The present invention relates to the use of nucleotide sequences of the ANGPTL1 and ANGPL2 (angiopoietin-like) genes, the amino acid sequences of the proteins encoded thereby, and derivatives (e.g., fragments) or analogs thereof for the treatment and diagnosis of blood-related disorders and defects in vasculature. In specific embodiments, the ANGPTL1 and ANGPL2 proteins are of human origin.

### BACKGROUND OF THE INVENTION

Receptor tyrosine kinases (RTKs) belong to the family of transmembrane receptors that, upon phosphorylation of specific tyrosine residues following ligand activation of the receptor, trigger activation of second messenger signaling systems to induce a biological response. A number of receptors that are found within this superfamily are those involved in normal vascular and hematopoietic development, such as flk1/KDR, flt1 flt4, TIE1 and TIE2 (Termann et al., 1992; Endo et al., 2001; Shibuya et al., 1990; de Vries et al., 1992; Millauer et al., 1993; Galland et al., 1993). More specifically, flk1 and fit belong to the VEGF receptor family of endothelium-specific receptor tyrosine kinases. In mice, flk 1 and fit 1 are expressed early in yolk sac mesoderm at the time of blood island formation, and in endothelial cells of later embryos and adults (Brier et al., 1995; Fong et al., 1995; Shalaby et al., 1995). Flt4, a receptor related to fltl, is also expressed in embryonic endothelial cells and later becomes restricted to lymphatic vessels (Kaipainen et al., 1995).

TIE1 and TIE2 belong to the family of receptor tyrosine kinases with immunoglobulins and epidermal growth factor homology domains, whose homologues have been cloned in other species: murine TEK and zebrafish Z tie-1 and -2 (Davis et al., 1996; Maisonpierre et al., 1997; Neufeld et al., 1999; Valenzuela et al., 1999; Lyons et al., 1998). Expression of these receptors is restricted to differentiated vascular endothelial cells, as well as a subset of hematopoietic stem cells (Mustonen and Alitalo, 1995; Lyons et al., 1998; Claesson-Welsh, 1999; Hamaguchi et al., 1999).

Several members of the angiopoietin (ANGPTs or ANGs) family of proteins have been shown to interact with TIE2 receptor, either as agonists or antagonists (Davis et al., 1996; Maisonpierre et al., 1997; Valenzuela et al., 1999; Pham et al., 2001), to mediate remodeling of primary vascular networks into large blood vessels, a process termed angiogenesis (Risau, 1997; Liang et al., 2001). Not surprisingly, ANGs and TIE2 receptor have also been implicated in playing a critical role in promoting proliferation of hematopoietic progenitor cells (Takakura et al., 1998; Huang et al., 1999; Hattori et al., 2001).

Recently, several angiopoietin-related or -like proteins have been identified based on their structural homology to the angiopoietins family of proteins (Conklin et al., 1999; Kim et al., 1999a; Kim et al., 199b); angiopoietin-like 1 (ANGPTL1), angiopoietin-like or -related 2 (ANGPTL2 or ARP2), angiopoietin-like or -related 3 (ANGPTL3 or ARP3), angiopoietin-like 4 (ANGPTL4) and angiopoietin-like 5 (ANGPTL5).

Both ANGPTL1, formally angiopoietin-3 (Kim et al., 1999a), and ANGPTL2 (Kim et al., 1999b) molecules include the characteristic coiled-coil (α-helical rich) segment, which helps to multimerize the fibrinogen-like domain; both domains are conserved in the angiopoietin family of proteins. The fibrinogen-like domain is a highly conserved receptor-binding domain, which determines whether an angiopoietin molecule will behave as an agonist or antagonist (Xu et al., 1996; Procopio et al., 1999). The gene expression patterns for ANGPTL1 and ANGPTL2 differ in human adult tissue: ANGPTL1 is ubiquitously expressed (Kim et al., 1999a) whereas ANGPTL2 is expressed specifically in the spleen, stomach, heart and small intestine. Furthermore, ANGPTL1 is highly expressed in vascularized human adult tissues (Kim et al., 1999a).

Mitogenic assays for ANGPTL1 in human umbilical vein endothelial cells (HUVECs) show that ANGPTL1 protein is secreted by endothelial cells, however, it cannot stimulate endothelial cell proliferation (Kim et al., 1999a), suggesting that this mitogen is only required for the maintenance of differentiated stage of endothelial cells in most human adult tissue. Furthermore, recombinant ANGPTL2 has been shown to induce sprouting of cultured human umbilical vein endothelial cells (HUVECs) (Kim et al. 1999b), but it also does not stimulate endothelial cell proliferation. This effect has also been observed for angiopoietin-1 (ANG1), an agonist of the T1E2 receptor (Davis et al., 1996). Since ANGPTL1 and ANGPTL2 hold a significant degree of amino acid sequence homology to ANG1, it is possible that both mitogenic factors induce sprouting of endothelial cells by activating the phophatidylinositol 3'-kinase/Akt signal transduction pathway via the TIE receptors (Kontos et al., 1998; Kim et al., 2000). Furthermore, ANG1 has recently been shown to be an apoptosis survival factor for endothelial cells (Kwak et al., 1999), suggesting that ANGPTL1, and possibly ANGPTL2 may also be an apoptosis survival factor for endothelial cells during vascular remodeling, similar to that of ANG1. However, neither of these molecules (ANGPTL1 and ANGPTL2) has been shown to bind TIE1 or TIE2 (Kim et al., 1999b), suggesting that a similar receptor/signal transduction pathway is being utilized to mediate vascular remodeling.

Furthermore, since TIE/TEK receptors are expressed in hemangioblasts (Hamaguchi et al., 1999), and both ANGs and TIE2 receptor have been implicated in playing a critical role in promoting proliferation of hematopoietic progenitor cells (Takakura et al., 1998; Huang et al., 1999; Hattori et al., 2001), angiopoietin-like proteins, via a similar receptor, may play a similar role in stimulating these multipotent cells to differentiate into hematopoietic stem cells during development.

### SUMMARY OF THE INVENTION

The present invention provides novel evidence for the role of the ANGPTL1 and ANGPTL2 genes in the cellular processes of erythroid growth, differentiation, maturation, and survival.

Generally, the present invention relates to the use of nucleotide sequences of the ANGPTL1 and ANGPTL2 (angiopoietin-like or related) genes, the amino acid sequences of their encoded proteins, and derivatives (e.g., fragments) or analogs thereof. In specific embodiments, the ANGPTL1 and ANGPTL2 proteins are human proteins.

In certain preferred embodiments, said nucleotide sequences used according to the present invention are operably linked to an expression control sequence. The invention also provides host cells, including bacterial, yeast, insect and mammalian cells, transformed with such nucleotide sequences.

The present invention provides a pharmaceutical composition comprising a polynucleotide sequence selected from the group consisting of:
a. the polynucleotide sequence of SEQ ID NO:2;
b. fragments and derivatives of the polynucleotide sequence of SEQ ID NO:2;
c. the polynucleotide sequence of SEQ ID NO:3;
d. fragments and derivatives of the polynucleotide sequence of SEQ ID NO:3.

The composition may further comprise a pharmaceutically acceptable carrier.

Compositions comprising a protein biological activity are also provided. In preferred embodiments the composition comprises an amino acid sequence selected from group consisting of:
(a) the amino acid sequence of SEQ ID NO: 7;
(b) fragments and derivatives of the amino acid sequence of SEQ ID NO: 7;
(c) the amino acid sequence of SEQ ID NO: 8;
(d) fragments and derivatives of the amino acid sequence of SEQ ID NO: 8; and the protein being substantially free from other mammalian proteins. Such compositions may further comprise a pharmaceutically acceptable carrier.

Methods are also provided for treating of blood-related disorders and defects in vasculature, which comprises administering to a mammalian subject a therapeutically effective amount of a composition comprising:
(a) a polypeptide selected from the group consisting of:
   a. the amino acid sequence of SEQ ID NO: 7;
   b. fragments or derivatives of the amino acid sequence of SEQ ID NO: 7;
   c. the amino acid sequence of SEQ ID NO: 8;
   d. fragments or derivatives of the amino acid sequence of SEQ ID NO: 8; and/or
(b) a polynucleotide sequence selected from the group consisting of:
   a. the polynucleotide sequence of SEQ ID NO: 2;
   b. fragments or derivatives of the polynucleotide sequence of SEQ ID NO: 2;
   c. the polynucleotide sequence of SEQ ID NO: 3;
   d. fragments or derivatives of the polynucleotide sequence of SEQ ID NO: 3.

The composition may further contain a pharmaceutically acceptable carrier.

Furthermore, the present invention relates to a method for screening a library of test molecules or compounds to identify at least one molecule or compound which specifically binds and/or interacts with polypeptides selected from the group consisting of:
(a) the amino acid sequence of SEQ ID NO: 7;
(b) fragments and derivatives of the amino acid sequence of SEQ ID NO: 7;
(c) the amino acid sequence of SEQ ID NO: 8;
(d) fragments and derivatives of the amino acid sequence of SEQ ID NO: 8; and the library of molecules or compounds is selected from the group consisting of DNA molecules, peptides, agonists, antagonists, antibodies, immunoglobulins and pharmaceutical agents.

In one aspect, the invention provides a pharmaceutical composition comprising angiopoietin-like 1 (ANGPTL1) or angiopoietin-like 2 (ANGPTL2) protein, a fragment or a derivative thereof.

Preferably, this composition comprises a polypeptide, which is at least 55% identical with SEQ ID NO: 7, or SEQ ID NO: 8, a fragment or derivative thereof.

Furthermore, the invention is directed to methods for treating a blood-related disorder or a defect in vasculature, comprising administering to a patient a therapeutically effective amount of polypeptide comprising ANGPTL1 or ANGPTL2, or a fragment or a derivative thereof. Preferably, the polypeptide is at least 55% identical with SEQ ID NO: 7 or SEQ ID NO: 8, fragment or derivative thereof.

A further aspect of the invention encompasses a pharmaceutical composition comprising a polynucleotide sequence encoding ANGPTL1 or ANGPTL2, a fragment, or a derivative thereof. Preferably, the polynucleotide sequence is at least 70% identical with a polynucleotide sequence which encodes SEQ ID NO: 7, or SEQ ID NO: 8, a fragment or a derivative thereof or the polynucleotide sequence is at least 70% identical with a polynucleotide sequence selected from the group comprising of SEQ ID NO: 2, or SEQ ID NO: 3. In a preferred embodiment the composition further comprises a suitable carrier. Preferably, the polynucleotide sequence is operably linked to an expression control sequence. In one embodiment of the invention the polynucleotide sequence, which is operably linked to an expression control sequence is in a host cell. This host cell is preferably a yeast, insect, teleost, amphibian or mammalian cell.

Furthermore, the invention is directed to methods for treating a blood-related disorder or a defect in vasculature, comprising administering to a patient a therapeutically effective amount of polynucleotide sequence encoding ANGPTL1 or ANGLPT2, or a fragment or a derivation thereof. Preferably, the polynucleotide sequence is at least 70% identical with a polynucleotide sequence which encodes SEQ ID NO:7 or SEQ ID NO:8, or a fragment or a derivative thereof or is at least 70% identical with a polynucleotide sequence selected from the group consisting of SEQ ID NO:2 or SEQ ID NO:3.

Preferably the polynucleotide sequence is in a delivery complex and the delivery complex can be a sterol, lipid or virus.

In a further aspect, the invention provides methods of screening for a therapeutic agent for the treatment of blood-related disorders or a defect in vasculature comprising: contacting ANGPTL1 polypeptide, ANGPTL2 polypeptide, fragment or derivative thereof with a test compound or molecule or a library of test molecules or compounds, under conditions to allow specific binding and/or interaction; and detecting the level of specific binding and/or interaction, wherein an alteration in the level of interaction relative to a control indicates a potential therapeutic activity.

Furthermore, the invention is directed to methods of screening for a therapeutic agent for the treatment of blood-related disorders or a defect in vasculature comprising: contacting a reporter construct under the control of ANGPTL1 or ANGPTL2 promoter with a test molecule or compound or a library of test molecules or compounds, under conditions to allow specific binding and/interaction; and detecting the level of expression of the reporter construct, wherein an alteration in the level of expression to a control indicates a potential therapeutic activity.

Preferably, the test molecule or test compound or library of test molecules or compounds is selected from the group consisting of DNA molecules, peptides, agonists, antagonists, monoclonal antibodies, immunoglobulins and pharmaceutical agents.

In a further embodiment the invention relates to methods for the treatment of blood-related disorders or defects in vasculature in a mammalian, preferably human patient comprising: providing a composition that comprises test molecules or compounds of therapeutic agents identified to bind or interact with angiopoietin-like polypeptides or modulate the level of angiopoietin-like expression; AND administering a therapeutically effective amount of said composition to the patient. Preferably, the composition comprising the test molecules or compounds also comprises a suitable carrier.

In a preferred embodiment, the blood-related disorder treated according to the methods of the present invention is leukemia or anemia.

A further aspect of the invention is a method of modulating proliferation and/or differentiation or cell death of target cells comprising administering a biologically active amount of ANGPTL1 or ANGPTL2 polypeptide or polynucleotide to said target cells. Preferably, said target cells are hematopoietic stem cells, erythroid or endothelial cells.

One embodiment of invention provides a method for determining whether a subject is at risk of developing or has a blood related disease or a defect in vasculature caused by ANGPTL1 or ANGPTL2 comprising determining the angiopoietin-like allele status of a subject; an altered angiopoietin-like allele status compared to a control indicating that said subject is at risk of developing or has a blood-related disease or a defect in vasculature caused by angiopoietin-like proteins.

Furthermore, the invention relates to a kit for identifying a subject at risk of developing a blood-related disease or a defect in vasculature or has a blood-related disease or a defect in vasculature caused by angiopoietin-like comprising means for measuring the level of an angiopoietin-like encoding polynucleotide sequence in a sample of cells of the subject and instructions to use the kit.

In another embodiment of the invention provides a transgenic non-human animal, which does not produce functional ANGPTL1 and/or ANGPTL2 protein or which produces sub-optimal levels of ANGPTL1 and/or ANGPTL2 protein or which overproduces the ANGPTL1 or ANGPTL2 protein. Preferably, the animal comprises a mutation of ANGPTL1 or ANGPTL2 genes. More preferably said mutation is a homozygous disruption. This mutation can be generated by insertion, deletion, or a substitution mutation. In a preferred embodiment said mutation is generated by gene targeting, gene trap integration, or mutagenesis. Preferably, the mutation has occurred in an exon, intron, regulatory region or splicing site of the ANGPTL1 and/or ANGPTL2 genes. In a further embodiment said animal exhibits a blood-related disease or a defect in vasculature and preferably said animal is a mammal, amphibian, teleost or insect.

The invention further provides antisense oligomers, wherein said antisense oligomesr are complementary to mRNA encoding ANGPTL1 or ANGPTL2, and are from 6 to 50 nucleotides in length. Preferably, the antisense oligomers are complementary to the translation initiation site of ANGPTL1 or ANGPTL2. More preferably said antisense oligomers are complementary to the polynucleotide sequence of SEQ ID NO: 2, SEQ ID NO: 3, or SEQ ID NO: 4, or the antisense oligomer comprises the nucleotide sequence SEQ ID NO: 5 or SEQ ID NO: 6. In a preferred embodiment the binding of said oligomer to said mRNA is effective in altering transcription or translation of said mRNA in a host cell expressing said mRNA. Preferably, the host cell is a bacterial, yeast, insect, or vertebrate cell. Furthermore, the antisense oligomer is preferably in a delivery complex and said delivery complex can be a sterol, lipid, or virus.

In one preferred embodiment the oligomers comprise: a) at least one modified base moiety which is selected from the group of 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5oxyacetic acid methylester, uracil-5-oxyacetic acid (v), -5-methyl-2-thiouracil, 3-(3-amino-3-N-2-carboxypropyl) uracil, (acp3)w, and 2,6-diaminopurine, AND/OR at least one modified sugar moiety selected from the group including but not limited to arabinose, 2-fluoroarabinose, xylulose, and hexose, AND/OR at least one modified phosphate backbone selected from the group consisting of a phosphorothioate, a phosphoramidothioate, a phosphoramidate, a phosphordiamidate, a methylphosphonate, an alkyl phosphotriester, a formacetal or analog thereof, as well as α-DNA, 2'-O-methyl RNA, and morpholine backbone.

### DECRIPTION OF FIGURES

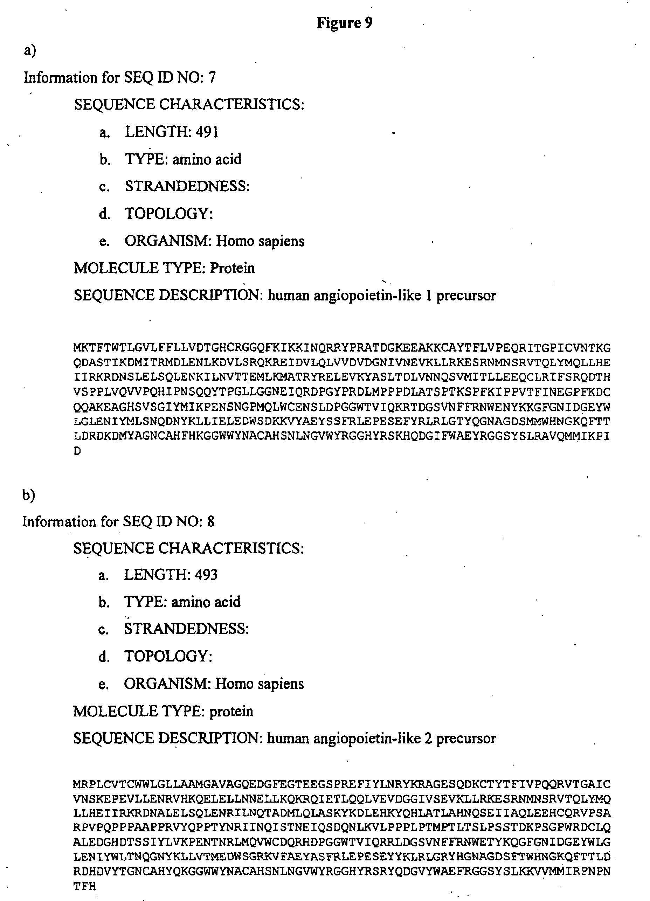

Figure 1. Nucleotide sequence of zfEST encoding a partial polypeptide belonging to the angiopoietin Y1 of proteins.
Figure 2. Results of BLASTP search using longest ORF of zfEST (AW174181)
Figure 3. Multiple sequence alignment of vertebrate angiopoietin-like family proteins.

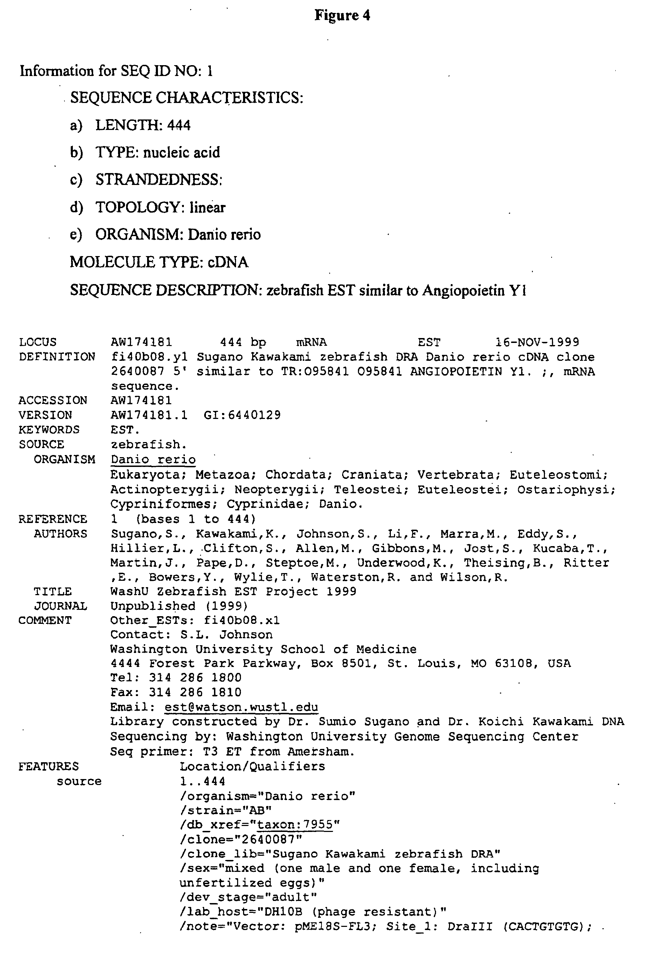

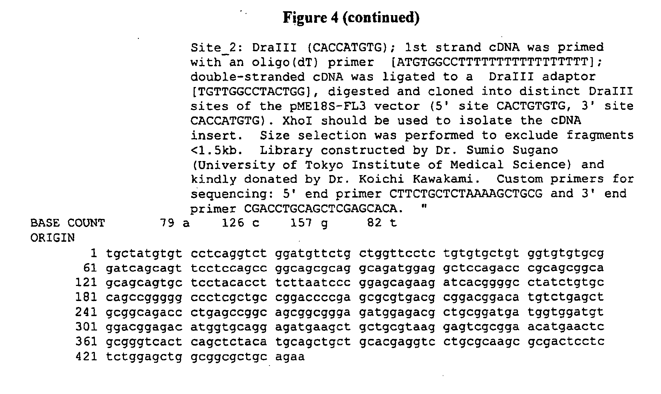

Figure 4. cDNA sequence of zebrafish cDNA clone similar to angiopoietin Y1.

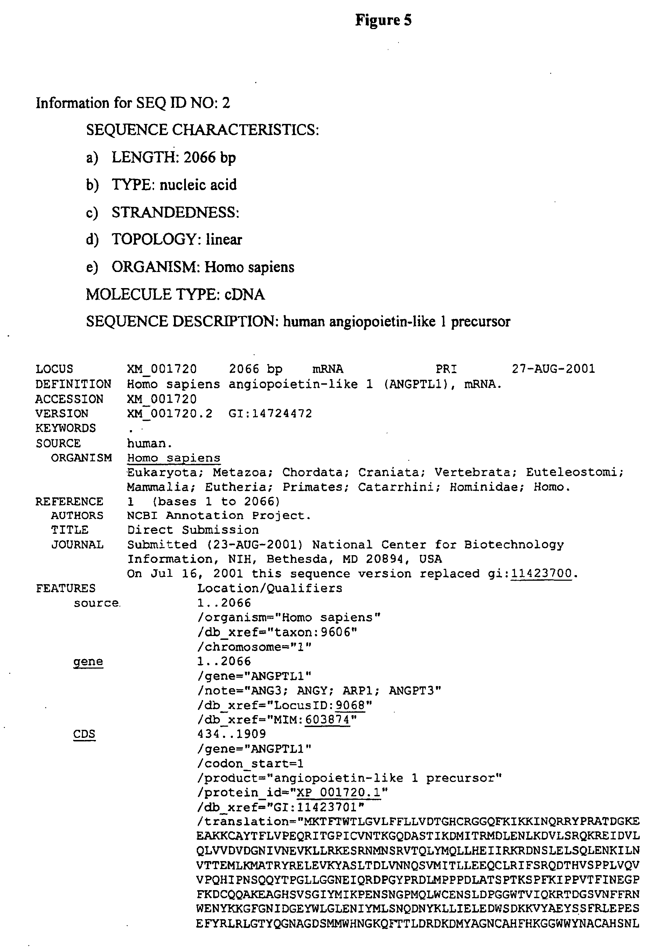

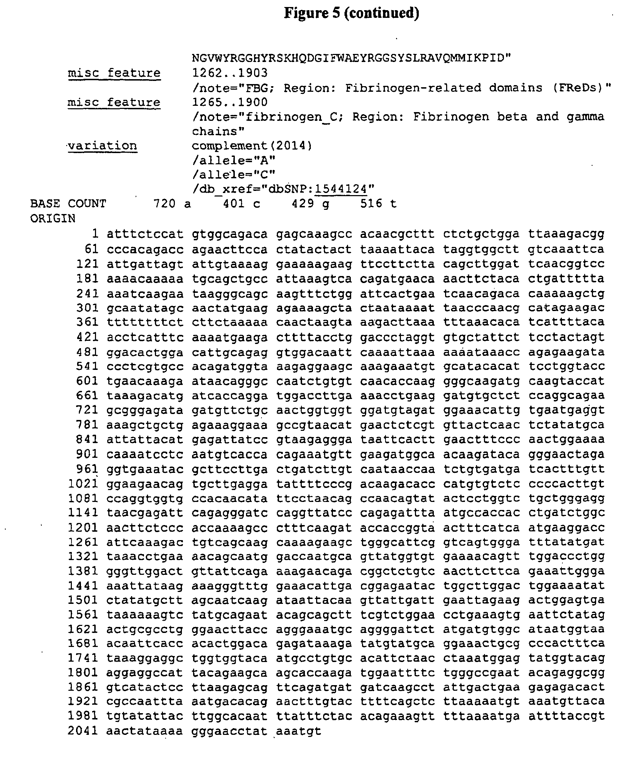

Figure 5. cDNA sequence of human angiopoietin-like 1 precursor.

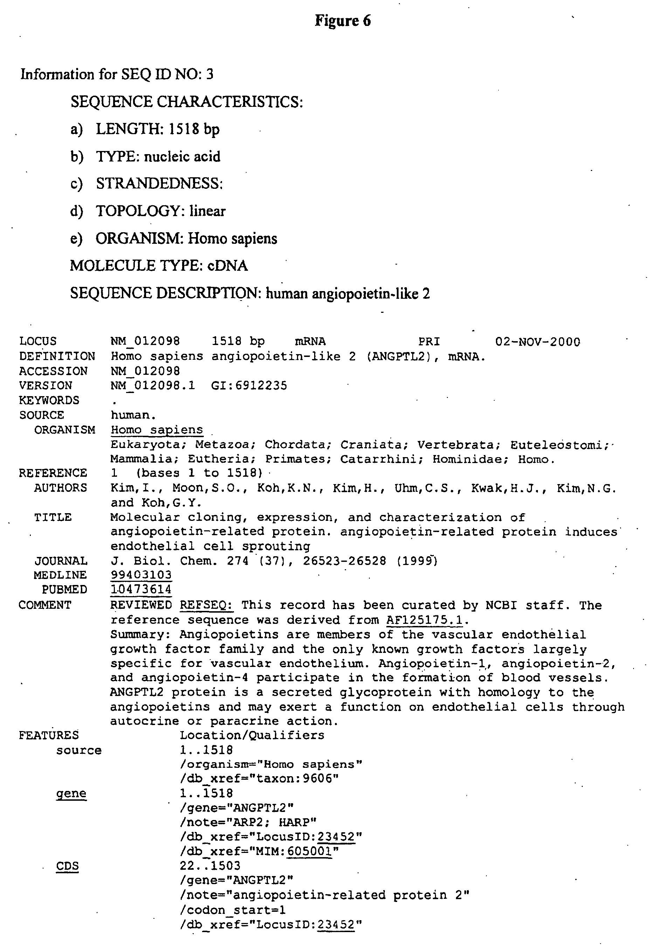

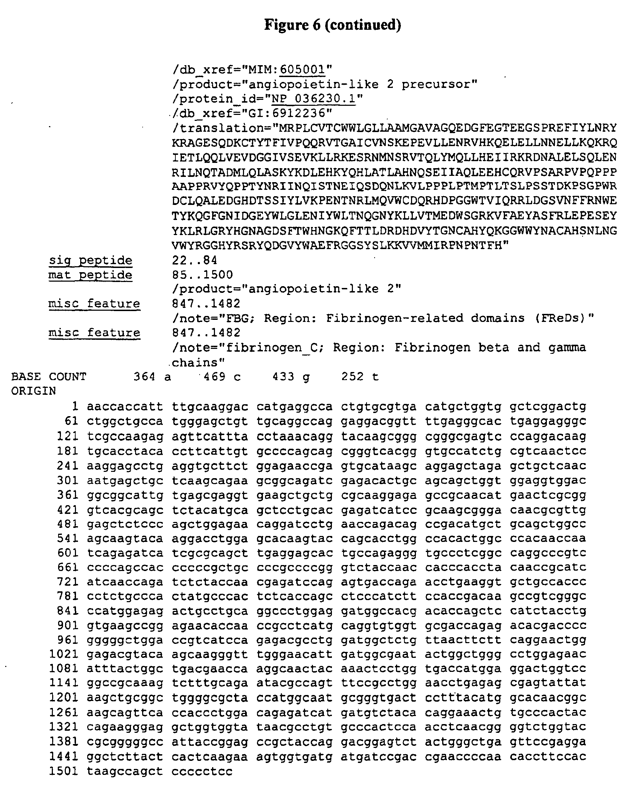

Figure 6. cDNA sequence of human angiopoietin-like 2 precursor.

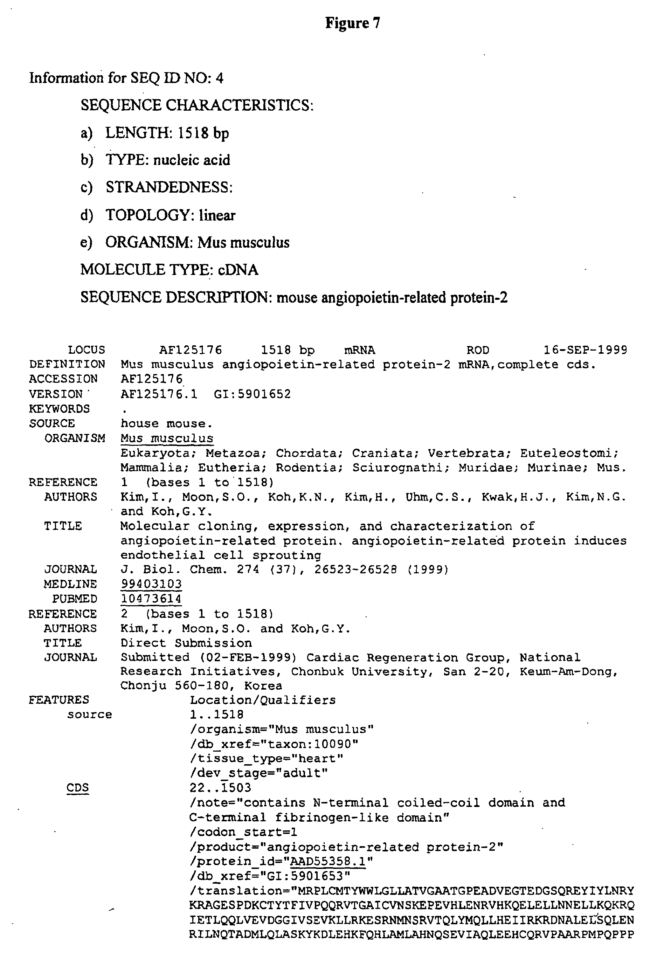

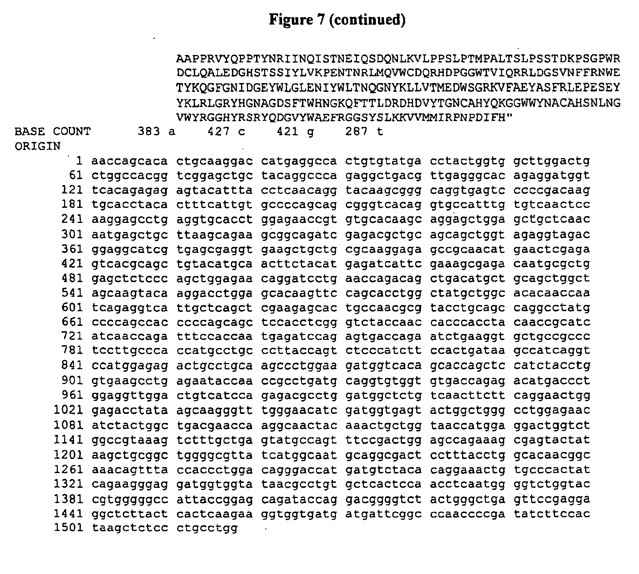

Figure 7. cDNA sequence of mouse angiopoietin-related 2 precursor.

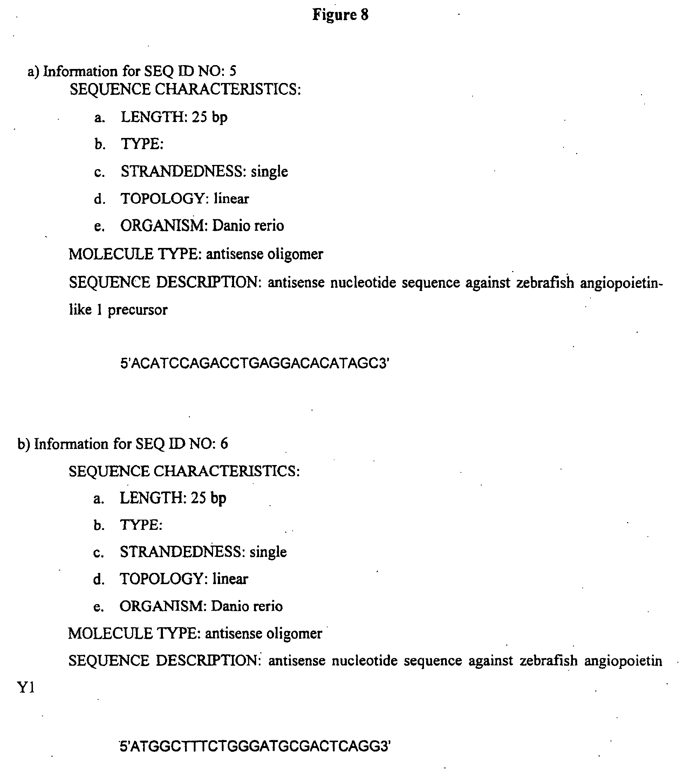

Figure 8. antisense oligonucleotide against zebrafish angiopoietin-like
   a) antisense oligonucleotide sequences against zebrafish angiopoietin-like 1 precursor
   b) antisense oligonucleotide sequences against zebrafish angiopoietin Y1
Figure 9. amino acid sequence for:
   a) human angiopoietin-like 1 precursor.
   b) human angiopoietin-like 2 precursor.

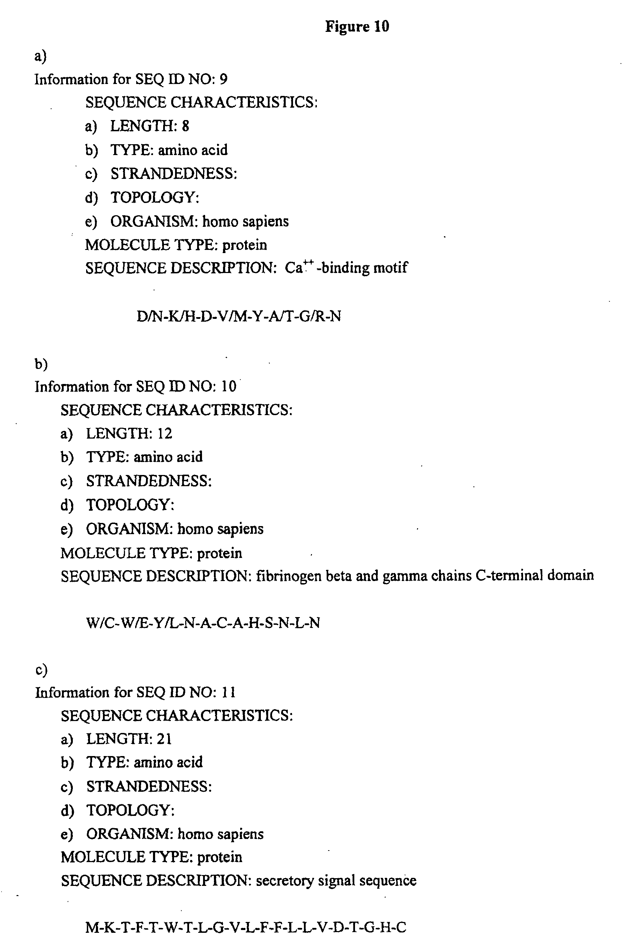

Figure 10. amino acid sequences of motifs found in angiopoietin-like molecules:
   a) The amino acid sequence of the calcium-binding motif,
   b) The amino acid sequence of the fibrinogen beta and gamma chains C-terminal domain,
   c) The amino acid sequence of the secretory signal sequence.
Figure 11. Analysis of hemoglobin (o-dianisidine) stained zebrafish embryos after morpholino (MO) knockdown.
Figure 12. Whole mount in situ hybridization analysis
   a) GATA-1 marker expression
   b) Flt-4 marker expression
   c) Flk-1 marker expression
   d) Fli-1 marker expression
   e) lmo-2 marker expression

### DETAILED DESCRIPTION

It is to be understood that the present invention is not limited to the particular materials and methods described or equipment, as these may vary. It is also to be understood that the terminology used herein is for the purpose of describing particular embodiments only, and is not intended to limit the scope of the present invention, which will be limited only by the appended claims.

It should be noted that as used herein and in the appended claims, the singular forms "a," "an," and "the" include plural reference unless the context clearly dictates otherwise. Thus, for example, a reference to "a host cell" includes a plethora of such host cells, and a reference to "an antibody" is a reference to one or more antibodies and equivalents thereof known to those skilled in the art, and so forth.

Unless defined otherwise, all technical and scientific terms used herein have the same meanings as commonly understood by one of ordinary skill in the art. Although any materials and methods, or equipment similar or equivalent to those specifically described herein can be used to practice or test the present invention, the preferred equipment, materials and methods are described below. All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

### DEFINITIONS

The term "derivative" refers to the chemical modification of a polypeptide sequence, a polynucleotide sequence, or antisense oligonucleotide sequence. Chemical modifications of a polynucleotide sequence include, but are not limited to, for example, the replacement of hydrogen by an alkyl, acyl, or amino group.

The term "biologically active," is used interchangeably with the terms "biological activity", "bioactivity" or "activity" and for the purposes herein means an effector or antigenic function that is directly or indirectly performed by an angiopoietin-like polypeptide (whether in its native or denatured conformation), or by any subsequence thereof. Effector functions include phosphorylation (kinase activity) or activation of other molecules, induction of differentiation, mitogenic or growth promoting activity, induction of apoptosis, signal transduction, immune modulation, DNA regulatory functions and the like, whether presently known or inherent. Antigenic functions include possession of an epitope or antigenic site that is capable or cross-reacting with antibodies raised against a naturally occurring or denatured angiopoietin-like polypeptide or fragment thereof. Accordingly, a biological activity of an angiopoietin-like protein can be phosphorylation (kinase activity) of other molecules. A biological activity of an angiopoietin-like protein can also be modulation of cell proliferation and/differentiation. A target cell can be, e.g., hematopoietic stem cell, erythroid progenitor cell, an endothelial cell, or a cancer cell.

A "composition comprising a given nucleotide sequence" or a "composition comprising a given amino acid sequence" refer broadly to any composition containing the given polynucleotide or amino acid sequence (polypeptide). The composition may comprise a dry formulation or an aqueous solution. Compositions comprising polynucleotide sequences encoding ANGPTL or fragments of ANGPTL may be employed as hybridization probes. The probes may be stored in freeze-dried form and may be associated with a stabilizing agent such as a carbohydrate. In hybridizations, the probe may be deployed in an aqueous solution containing salts (e.g., NaCI), detergents (e.g., sodium dodecyl sulfate; SDS), and other components (e.g., Denhardt's solution, dry milk, salmon sperm DNA, etc.).

A coding sequence is "operably linked to" another coding sequence when RNA polymerase will transcribe the two coding sequences into a single mRNA which is then translated into a single polypeptide having amino acids derived from both coding sequences. The coding sequences need not be contiguous to one another so long as the expressed sequence is ultimately processed to produce the desired protein. The term "reporter construct" encompasses a target gene linked in frame to another sequence to provide a coding unit whose product is easily assayed. Examples of reporter genes include neo, geo, hyro and puro.

The phrases "nucleotide sequence", "oligonucleotide sequence" or "polynucleotide sequence" refer to a nucleotide, oligonucleotide, polynucleotide, or any fragment thereof. These phrases also refer to DNA or RNA of genomic or synthetic origin which may be single-stranded or double-stranded and may represent the sense or the antisense strand, to peptide polynucleotide sequence (PNA), or to any DNA-like or RNA-like material. In this context, "fragments" refers to those polynucleotide sequence sequences which, when translated, would produce polypeptides retaining some functional characteristic, e.g., antigenicity, or structural domain characteristic, e.g., ATP-binding site, of the full-length polypeptide.

As is well known, genes for a particular polypeptide may exist in single or multiple copies within the genome of an individual. Such duplicate genes may be identical or may have certain modifications, including nucleotide substitution, additions or deletions, all of which still code for polypeptides having substantially the same activity. The term "polynucleotide sequence encoding an angiopoietin-like polypeptide" may thus refer to one or more genes within a particular individual. Moreover, certain differences in nucleotide sequences may exist between individual organisms, which are called alleles. Such allelic differences may or may not result in differences in the amino acid sequence of the encoded polypeptide yet still encode a protein with the same biological activity.

The term "oligomer/s" is used herein to mean oligonucleotides and related structural compounds.

The term "transcriptional regulatory sequence" or "regulatory region" or "expression control sequence" includes promoters, enhancers and other expression control elements.

"Cells", "host cells", "target cells" or "recombinant host cells" are terms used interchangeably herein and refer to a cell which has been transformed or transfected, or is capable of transformation or transfection by an exogenous polynucleotide sequence. It is understood that such terms refer not only to the particular subject cell but also to the progeny or potential progeny of such a cell. Because certain modifications may occur in succeeding generations due to either mutation or environmental influences, such progenys may not, in fact, be identical to the parent cell, but are still included within the scope of the term as used herein.

As used herein, a "target molecule" is a molecule with which said polypeptide binds or interacts with, for example, a molecule associated with the internal surface of a cell membrane or a cytoplasmic molecule.

A "delivery complex" shall mean a targeting means (e.g., a molecule that results in higher affinity binding of an antisense oligomer, protein, polypeptide, or peptide to a target cell surface and /or increased cellular uptake by a target cell). Examples of targeting means include: sterols (e.g. cholesterol), lipids (e.g. a cationic lipid, virosome or liposome), viruses (e.g. adenovirus, adeno-associated virus, or retrovirus). Preferred complexes are sufficiently stable *in vivo* to prevent significant uncoupling prior to internalization by the target cell. However, the complex is cleavable under appropriate conditions within the cell so that the polynucleotide, protein, polypeptide, or peptide is released in a functional form.

"A blood-related disorder" encompasses any disease, ailment, or malady that is the result of defects during hematopoiesis, erythropoiesis, hemoglobin synthesis and/or heme production. These include, but are not limited to, a failure of hematopoietic stem cells, erythroid-myeloid progenitor cells or lymphoid progenitor cells to differentiate; abnormal differentiation of hematopoietic stem cells, erythroid-myeloid progenitor cells or lymphoid progenitor cells; absence or decreased production of hemoglobin; or inhibited heme synthesis.

"A defect in vasculature" relates to an alteration in normal blood vessel formation within any given animal. In the context of the invention, this includes, but is not limited to, a lack or significant decrease of blood vessel formation, an overabundance of blood vessels, invasive blood vessel formation (amount of vessels formed is normal, however formation follows no defined pattern), abnormally slow or rapid (e.g. hemangionomas) vasculogenesis and/or angiogenesis, arrested vasculogenesis and/ or angiogenesis, or abnormal blood vessel wall formation (e.g. structural defects).

"Antisense oligomer" refers to an antisense molecule or anti-gene agent that comprises an oligomer of at least about 5 nucleotides, (or any derivatives thereof) in length. In embodiments an antisense oligomer comprises at least 6, 7, 8, 9, 10, 12, 15, 18 or 20 nucleotides.

A "transgenic animal" refers to any animal, preferably a non-human mammal, bird, fish or an amphibian, in which one or more of the cells of the animal contain heterologous polynucleotide sequence introduced by way of human intervention, such as by transgenic techniques well known in the art. The polynucleotide sequence is introduced into the cell, directly or indirectly by introduction into a precursor of the cell, by way of deliberate genetic manipulation, such as by microinjection or by infection with a recombinant virus. This polynucleotide sequence may be integrated within a chromosome (stable transgenic), or it may be extra-chromosomally replicated (transient transgenic). In the typical transgenic animals described herein, the transgene causes cells to express a recombinant form of one of the angiopoietin-like proteins, e.g. either agonistic or antagonistic forms. Moreover, "transgenic animal" also includes those recombinant animals in which gene disruption of one or more angiopoietin-like genes is caused by human intervention, including both recombination and antisense techniques.

The term "does not produce functional ANGPTL1 and/or ANGPTL2 protein" denotes the lack of translation of an effective ANGPTL1 and/or ANGPTL2 protein.

The term "produces sub-optimal levels of ANGPTL1 and/or ANGPTL2" encompasses the translation level of ANGPTL1and/or ANGPTL2 protein, which is insufficient to produce an effective concentration of ANGPTL1 and/or ANGPTL2. Preferably, the level is reduced at least by 50%, more preferably by 70% and most preferably by 95 %.

The term "homozygous disruption of the ANGPTL1 and/or ANGPTL2 genes" relates to an identical mutation in both ANGPTL1 and/or ANGPTL2 alleles.

The term "mutation" refers to a change of one or more nucleotide pairs of a DNA molecule.

The term "insertion" is directed to a mutation identified by the presence of one or more additional base pairs in the DNA.

The term "deletion" relates to a mutation generated by removal of a sequence of DNA (one or more base pairs), the regions on either side being joined together.

The term "substitution mutation" is directed to a nucleotide change. The substitution mutation can result in an amino acid change or can introduce a premature translation stop codon. Furthermore, a substitution mutation can affect splicing or expression of the gene when occurring in the sites necessary for splicing or regulatory regions of the gene.

The term "gene targeting" relates to a type of homologous recombination that occurs when a fragment of genomic DNA is introduced into a cell and that fragment locates and recombines with homologous sequences.

The term "gene trap integration" is directed to insertion of a vector, which comprises a reporter gene and which is activated upon its insertion within an active transcription unit, into a chromosomal site.

The term "mutagenesis" denotes a chemical or physical treatment that changes nucleotides in the genome of an organism. An example of a chemical mutagenesis is N-ethyl-N-nitrosurea (ENU) mutagenesis.

The term "exon" encompasses a segment of a gene which is decoded to give a mRNA product or a mature RNA product. Individual exons may contain protein coding DNA and/or non-coding DNA.

The term "intron" denotes non-coding DNA, which separates neighboring exons in a gene. During gene expression introns, like exons are transcribed into RNA but the transcribed intron sequences are subsequently removed by RNA splicing and are not present in mRNA.

The term "regulatory region" relates to the nucleotide sequence, which comprises regions, which are necessary for the regulation of the transcription of the gene. These regions comprise e.g. promoters and enhancers and they can be located in the 5' region of the exons or in introns.

The term "splicing site" encompasses the nucleotides at a) the end of an exon and the beginning of the intron and at b) the end of the intron and beginning of the exon that are required for the joining of two exons and removal of an intron during the processing of a primary transcript to a functional mRNA.

The term "angiopoietin-like gene allele status" denotes the state of the DNA sequence in both alleles of an angiopoietin-like gene, i.e. whether the angiopoietin-like gene contains a mutation and whether this mutation is heterozygous or homozygous.

The terms "specific binding" or "specifically binding" refer to the interaction between two proteins, a protein and a peptide, two peptides, a protein or peptide and an agonist, an antibody, or an antagonist. The interaction is dependent upon the presence of a particular structure of the protein, e.g., docking site of specific kinases, phosphorylated amino acids, the antigenic determinant or epitope, recognized by the binding molecule. For example, if the amino acid sequence of protein A docking site is recognized by the protein docking site of protein B; in a reaction containing protein A, labeled protein B and unlabeled protein B, the unlabeled protein B will reduce the amount of labeled protein B being able to bind protein A. The term "interaction" relates to direct or indirect binding or alteration of expression at the polynucleotide or polypeptide level.

The term "agonist" refers, in one embodiment, to a molecule which, when bound to ANGPTL, increases or prolongs the duration of the effect of angiopoietin-like proteins. Agonists may include proteins, polynucleotide sequences, carbohydrates, or any other molecules which bind to and modulate the effect of angiopoietin-like proteins.

The term "antagonist" refers, in one embodiment, to a molecule or compound which, when bound to ANGPTL1 and/or ANGPTL2 decreases receptor-binding activity of an angiopoietin-like protein. A preferred angiopoietin-like antagonist inhibits the interaction between an angiopoietin-like protein and another molecule, e.g. (tyrosine kinase receptors). Alternatively, an angiopoietin-like antagonist can be a compound which modulates the expression or activity of a protein which is located downstream of angiopoietin-like or which interacts with an angiopoietin-like protein. An angiopoietin-like antagonist can also be a molecule or compound which decreases the amount or the duration of the effect of the biological or immunological activity of angiopoietin-like proteins. A preferred angiopoietin-like antagonist inhibits the interaction between an angiopoietin-like protein and another molecule, e.g. tyrosine kinase receptors. Antagonists may include proteins, polynucleotide sequences, carbohydrates, antibodies, or any other molecule that exerts these effects.

The term "stringent conditions" refers to conditions which permit hybridization between polynucleotides and the claimed polynucleotides. Stringent conditions can be defined by salt concentration, the concentration of organic solvent, e.g., formamide, temperature, and other conditions well known in the art. In particular, stringency can be increased by reducing the concentration of salt, increasing the concentration of formamide, or raising the hybridization temperature.

A "chimeric protein" or "fusion protein" is a fusion of a first amino acid sequence encoding an angiopoietin-like polypeptide with a second amino acid sequence defining a domain (e.g. polypeptide portion) foreign to and not substantially homologous with any domain of the angiopoietin-like protein.

A chimeric protein may present a foreign domain which is found (albeit in a different protein) in an organism which also expresses the first protein, or it may be an "interspecies", "intergenic", e.g. fusion of protein structures expressed by different kinds of organisms. In general, a fusion protein can be represented by the general formula X-ANGPTL-Y, wherein ANGPTL represents a portion of the protein which is derived from the angiopoietin-like protein, and X and Y are independently absent or represent amino acid sequences which are not related to one of the angiopoietin-like sequences in an organism, including naturally occurring mutants.

The term "biological sample" is intended to include tissues, cells and biological fluids isolated from a subject, as well as tissues, cells and fluids present within a subject.

### DETAILED DESCRIPTION OF THE INVENTION

### Angiopoietin-like protein therapeutics

The first aspect of the present invention provides pharmaceutical compositions comprising polypeptides selected from the group consisting of SEQ ID Nos. 7 or 8, fragments and derivatives thereof in combination with a pharmaceutically suitable carrier. In a preferred embodiment, therapeutically effective amounts of said polypeptides, fragments and derivatives will be used in the treatment of blood disorders and defects in vasculature. In an even more preferred embodiment, the blood-related disorders are leukemia and anemia.

In another embodiment of the invention, pharmaceutical compositions are provided comprising polynucleotides selected from the group consisting of SEQ ID Nos. 2 or 3, fragments and derivatives thereof, in combination with a pharmaceutically acceptable carrier. In one aspect, the complement of said polynucleotides may be used in situations in which it is desirable to block transcription of mRNA. In particular, cells may be transformed with sequences complementary to polynucleotides selected from SEQ ID Nos. 2 or 3, which encode angiopoietin-like proteins. Thus, complementary molecules or fragments may be used to modulate angiopoietin-like activity, or to achieve regulation of gene function. Such technology is well known to those skilled in the art. In a preferred embodiment, therapeutically effective amounts of said polynucleotides, fragments and derivatives will be used in the treatment of blood disorders and defects in vasculature. In an even more preferred embodiment, the blood-related disorders are leukemia and anemia.

Expression vectors derived from retroviruses, adenoviruses, or herpes or vaccinia viruses, or from various bacterial plasmids, may be used for delivery of polynucleotide sequences to the targeted organ, tissue (e.g. bone marrow), or cell population (hematopoietic stem cells, erythroid cells or endothelial cells). Methods which are well known to those skilled in the art, can be used to construct vectors to express nucleic acid sequences complementary to the polynucleotides encoding angiopoietin-like proteins (see Current Protocols In Molecular Biology, John Wiley & Sons, N.Y. 3.16.1-3.16.11).

An additional embodiment of the invention relates to the administration of a pharmaceutical composition, in conjunction with a pharmaceutically acceptable carrier, for any of the therapeutic effects discussed above. Such pharmaceutical compositions may comprise one or more agents selected from the group consisting of angiopoietin-like polypeptides, angiopoietin-like encoding polynucleotides, agonist and antagonists of angiopoietin-like proteins, inhibitors of angiopoietin-like proteins, and antibodies to angiopoietinlike proteins. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs, or hormones.

Pharmaceutical compositions used according to the present invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

For topical or nasal administration, penetrants appropriate to the particular barrier to be permeated are used in the formulation. Such penetrants are generally known in the art.

In a preferred embodiment, pharmaceutical compositions suitable for use in the invention include compositions wherein the active ingredients are contained in an effective amount to achieve the intended purpose. The determination of a therapeutically effective dose is well within the capability of those skilled in the art. For any pharmaceutical composition, the therapeutically effective dose can be estimated initially either in cell culture assays, e.g., leukemia cell lines, or in animal models such as zebrafish, mice, rats, or rabbits. An animal model may also be used to determine the appropriate concentration range and route of administration. Such information can then be used to determine useful doses and routes for administration in humans.

A therapeutically effective dose refers to that amount of active ingredient, for example angiopoietin-like polypeptides, angiopoietin-like encoding polynucleotides, agonist and antagonists of angiopoietin-like proteins, inhibitors of angiopoietin-like proteins, or antibodies to angiopoietin-like proteins, which ameliorates the symptoms or condition. Therapeutic efficacy and toxicity may be determined by standard pharmaceutical procedures in cell cultures or with experimental animals, known to those skilled in the art. Pharmaceutical compositions which exhibit large therapeutic indices are preferred.

The pharmaceutical composition of the present invention may be manufactured in a manner that is known to those skilled in the art, e.g. by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, and succinic acid. Salts tend to be more soluble in aqueous or other protonic solvents than are the corresponding free base forms.

### Screening for therapeutics

The invention provides a method, also referred to herein as a "screening assay", for identifying candidates, test compounds, test molecules, or agents (e.g. peptides, peptidomimetics, small molecules or other drugs) which bind to a polypeptide selected from the group consisting of SEQ ID Nos. 7 or 8, fragment or derivative thereof, or have a stimulatory or inhibitory effect on, for example, expression or activity of said polypeptides.

In one embodiment, the above-mentioned polypeptide has at least one bioactivity of an angiopoietin-like polypeptide, such as the ability to activate tyrosine kinase receptors. Proteins or peptides capable of interacting directly or indirectly with the angiopoietin-like proteins, fragments or derivatives thereof, can be identified by various methods, e.g., methods based on binding assays (see references on: yeast-2-hybrid Bemis et al. (1995) Methods Cell. Biol. 46:139-51, Fields et al. (1994) Trends Genet. 10:286-92, Topcu and Borden (2000) Pharm. Res. 17:1049-1055; yeast 3 hybrid: Zhang et al. (1999) Methods Enzymol. 306:93-113; GST-pull-downs as in Palmer et al. (1998) EMBO J. 17:5037-5047; and phage display as in Scott and Smith (1990) Science. 249:386-390).

In other preferred embodiments, the bioactivity of an angiopoietin-like polypeptide is to modulate proliferation and/or differentiation or cell death of specific target cells, e.g., hematopoietic stem cells, erythroid cells or endothelial cells. Assays for determining that an angiopoietin-like polypeptide has at least one bioactivity of an angiopoietin-like protein are described infra.

In one embodiment, the invention provides assays for screening test compounds or molecules which bind to or modulate the activity of the biologically active form of a polypeptide selected from the group consisting of SEQ ID Nos. 7 and 8, fragments and derivatives thereof. The test compounds according to the present invention can be obtained using any of the numerous approaches in combinatorial library methods known in the art, including: biological libraries, aptially addressable parallel solid phase or solution phase libraries, synthetic library methods requiring deconvolution, the "one-bead-one-compound" library method, and synthetic library methods using affinity chromatography selection. The biological library approach is limited to peptide libraries, while the other four approaches are applicable to peptide, non-peptide oligomer or small molecule libraries of compounds (Bindseil et al. (2001) Drug Discov. Today 6:840-847; Grabley et al. (2000) Ernst Schering Res. Found. Workshop. pp. 217-252; Houghten et al. (2000) Drug Discov. Today 5:276-285; and Rader (2001) Drug Discov. Today 6:36-43).

Examples of methods for the synthesis of molecular libraries can be found in the art, for example in: DeWitt et al. (1993) Proc. Natl. Acad. Sci. USA 90:6909-13; Erb et al. (1994) Proc. Natl. Acad. Sci. USA 91:11422-25; Gallop et al. (1994) J. Med. Chem. 37:1233-1251; and Gordon et al. (1994) J. Med. Chem. 37:1385-401.

Libraries of compounds may be presented in solution (e.g., Houghten (1992) Biotechniques. 13:412-421), or on beads (Lam (1991) Nature. 354:82-84), chips (Fodor (1993) Nature. 364:555-556), bacteria (U. S. Patent No. 5,223,409), spores (Patent NOS. 5,571,698; 5,403,484; and 5,223,409), plasmids (Cull et al. (1992) Proc. Natl. Acad. Sci. USA. 89:1865-1869) or phage (Scott and Smith (1990) Science. 249:386-390; Devlin (1990) Science. 249:404-406; Cwirla et al. (1990) Proc. Natl. Acad. Sci. USA. 87:6378-6382; and Felici (1991) J. Mol. Biol. 222:301-310).

In one embodiment, the assay is a cell-based assay in which a cell expresses a biologically active polypeptide selected from the group consisting of SEQ ID Nos. 7 or 8, a fragment or derivative thereof. The expressed polypeptide is contacted with a test compound and the ability of the test compound to bind to the polypeptide is determined. The cell can, for example, be a yeast cell, insect cell, teleost cell, an amphibian cell, or a cell of mammalian origin. Determining the ability of the test compound or molecule to bind to the polypeptide can be accomplished, for example, by coupling the test compound or test molecule with a radioisotope (e.g. ¹²⁵I, ³⁵S, ¹⁴C, or ³H) or enzymatic (e.g. horseradish peroxidase, alkaline phosphatase, or luciferase) label such that binding of the test compound or molecule to the biologically active polypeptide, fragment, or derivative thereof can be determined by detecting the labelled compound in the complex. Methods of labelling and detecting interactions of test compounds with cytosolic proteins are known to those skilled in the art. In a preferred embodiment, the assay comprises contacting a cell, which expresses a biologically active polypeptide selected from the group consisting of SEQ ID Nos. 7 or 8, fragment, or derivative thereof, with a known compound which binds the polypeptide to form an assay mixture, contacting the assay mixture with a test compound, and determining the ability of the test compound to interact with said polypeptide, wherein determining the ability of the test compound to interact with said polypeptide is compared to a control.

In another embodiment, the assay is a cell-based assay comprising contacting a cell expressing a biologically active polypeptide selected from the group consisting of SEQ ID Nos. 7 or 8, fragment, or derivative thereof, with a test compound or test molecule and determining the ability of the test compound or molecule to modulate (e.g., stimulate or inhibit) the activity of said polypeptide. Determining the ability of the test compound or molecule to modulate the activity of said polypeptide can be accomplished, for example, by determining the ability of said polypeptide to bind to or interact with a target molecule.

Determining the ability of a biologically active polypeptide selected from the group consisting of SEQ ID Nos. 7 or 8, fragment or derivative thereof, to bind to or interact with a target molecule can be accomplished by one of the methods described above for determining direct binding. A target molecule can be a biologically active polypeptide selected from SEQ ID. Nos. 7 or 8, fragment, or derivative thereof, or another polypeptide or protein. For example, a target molecule can be a component of a signal transduction pathway which facilitates transduction of an extracellular signal, a second intercellular protein which has catalytic activity, a protein which regulates transcription of specific genes, or a protein that initiates protein translation. Determining the ability of a biologically active polypeptide selected from the group consisting of SEQ ID Nos. 7 or 8, fragment or derivative thereof, to bind to or interact with a target molecule can be accomplished by determining the activity of the target molecule. For example, the activity of the target molecule can be determined by detecting induction of a cellular second messenger of the target (e.g., intracellular Ca++, diacylglycerol, IP3, etc.), detecting catalytic/enzymatic activity of the target on an appropriate substrate, detecting the induction (via a regulatory element that may be responsive to said polypeptide) of a reporter gene operably linked to a nucleic acid encoding a detectable marker, e.g., luciferase, or detecting a cellular response, for example, cellular differentiation, or cell proliferation.

In yet another embodiment, the assay of the present invention is a cell-free assay comprising contacting a biologically active polypeptide selected from the group consisting of SEQ ID Nos. 7 or 8, a fragment or derivative thereof with a test compound or molecule, and determining the ability of the test compound or molecule to bind to said polypeptide. Binding of the test compound or molecule to said polypeptide can be determined either directly or indirectly as described above. In a preferred embodiment, the assay includes contacting said polypeptide with a known compound, which binds the polypeptide to form an assay mixture. The assay mixture is contacted with a test compound or molecule, and the determination of the ability of the test compound or molecule to interact with the polypeptide is based on competitive binding/inhibition kinetics of the test compound ormolecule and known compounds for said polypeptide. Methods of detecting of competitive binding of two molecules for the same target, wherein the target is a polypeptide selected from the group consisting of SEQ ID. Nos. 7 or 8, fragments or derivatives thereof are known to those skilled in the art.

In another embodiment, the assay is a cell-free assay comprising contacting a biologically active polypeptide selected from the group consisting of SEQ ID Nos. 7 or 8, fragment, or derivative thereof with a test compound or molecule, and determining the ability of the test compound or molecule to modulate (e.g., stimulate or inhibit) the activity of said polypeptide. Determining the ability of the test compound or molecule to modulate the activity of said polypeptide can be accomplished, for example, by determining the ability of said polypeptide to bind to a target molecule by one of the methods described above for determining direct binding. In an alternative embodiment, determining the ability of the test compound or molecule to modulate the activity of said polypeptide can be accomplished by determining the ability of said polypeptide to further modulate a target molecule.

In embodiments of the above assay methods of the present invention, it may be desirable to immobilize either the polypeptide selected from the group consisting of SEQ ID Nos. 7 or 8, fragment, or derivative thereof, or its target molecule to facilitate separation of complexed from uncomplexed forms of one or both of the proteins, as well as to accommodate automation of the assay. Binding of a test compound or molecule to said polypeptide, or interaction of said polypeptide with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase fusion proteins or glutathione-S-transferase fusion proteins can be adsorbed onto glutathione sepharose beads (Sigma Chemical; St. Louis, MO) or glutathione derivatized microtitre plates, which are then combined with the test compound or the test molecule and either the non-adsorbed target protein or a biologically active polypeptide selected from the group consisting of SEQ ID Nos. 7 or 8, fragment or derivative thereof. The mixture is then incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of the polypeptide can be determined using standard techniques.

Other techniques for immobilizing proteins on matrices can also be used in the screening assays of the invention. For example, either the biologically active polypeptide selected from the group consisting of SEQ ID Nos. 7 or 8, fragment or derivative thereof, or its target molecule can be immobilized utilizing conjugation of biotin and streptavidin.

In another embodiment, modulators of expression of an ANGPTL polypeptide are identified in a method in which a cell is contacted with a candidate compound and the expression of the selected mRNA or protein (i. e., the mRNA or protein corresponding to a biologically active polypeptide (SEQ ID Nos. 7 or 8; SEQ ID Nos. 2 or 3) in the cell is determined. In a preferred embodiment, the cell is an animal cell. Even more preferred, the cell can be derived from insects, fish, amphibians, mice, rats, or humans. The level of expression of the selected mRNA or protein in the presence of the candidate compound is compared to the level of expression of the selected mRNA or protein in the absence of the candidate compound. The candidate compound can then be identified as a modulator of expression of the ANGPTL polypeptide based on this comparison. For example, when expression of the selected mRNA or protein is greater (statistically significantly greater) in the presence of the candidate compound than in its absence, the candidate compound is identified as a stimulator of the selected mRNA or protein expression. Alternatively, when expression of the selected mRNA or protein is less (statistically significantly less) in the presence of the candidate compound than in its absence, the candidate compound is identified as an inhibitor of the selected mRNA or protein expression. The level of the selected mRNA or protein expression in the cells can be determined by methods described herein.

In another embodiment, an angiopoietin-like therapeutic can also be identified by using a reporter assay in which the level of expression of a reporter construct, under the control of an angiopoietin-like protein promoter, is measured in the presence or absence of a test compound. An angiopoietin-like promoter can be isolated by screening a genomic library with an angiopoietin-like cDNA which preferably contains the 5' end of the cDNA. A portion of the angiopoietin-like promoter, typically from about 50 to about 500 base pairs long is then cloned upstream of a reporter gene, e.g., a luciferase gene, in a plasmid. This reporter construct is then transfected into cells, e.g., erythroid cells or endothelial cells. Transfected cells are then distributed into wells of a multi-well plate and various concentrations of test compounds are added to the wells. After several hours of incubation, the level of expression of the reporter construct is determined according to methods known in the art. A difference in the level of expression of the reporter construct in transfected cells incubated with the test compound relative to transfected cells incubated without the test compound will indicate that the test compound is capable of modulating the expression of the angiopoietin-like protein gene and is thus an angiopoietin-like therapeutic.

In one embodiment of the invention, angiopoietin-like therapeutics can be used for treating various diseases, including blood diseases, diseases or conditions associated with defects in vasculature, specifically leukemia and anemia. In addition, based at least in part on the similarity of the amino acid sequence and structure between the various angiopoietin-like proteins, angiopoietin-like therapeutics can be used to treat diseases or conditions associated with an aberrant angiopoietin-like activity or an aberrant angiopoietin-like activity other than angiopoietin-like activity.

This invention further pertains to novel agents identified by the above-described screening assays and uses thereof for treatments as described herein.

### Diagnostics

Another aspect of the present invention relates to diagnostic assays for determining the expression of an angiopoietin-like polypeptide or polynucleotide encoding an angiopoietin-like protein, and/or the activity of said polypeptide, in the context of a biological sample (e.g., blood, serum, cells, tissue) to thereby determine whether an individual is afflicted with a disease or disorder, or is at risk of developing a disorder, associated with aberrant expression or activity of an angiopoietin-like polypeptide, e.g. a blood-related disorder or a defect in vasculature. The invention also provides for prognostic (or predictive) assays for determining whether an individual is at risk of developing a disorder associated with aberrant expression or activity of an angiopoietin-like polypeptide. For example, mutations in a gene encoding an angiopoietin-like protein can be assayed in a biological sample. Such assays can be used for prognostic or predictive purpose to thereby prophylactically treat an individual prior to the onset of a disorder characterized by or associated with aberrant expression or activity of an angiopoietin-like polypeptide.

A method for detecting the presence or absence of an angiopoietin-like polypeptide or polynucleotide encoding an angiopoietin-like polypeptide, e.g., the amino acid sequences selected from the group consisting of SEQ ID Nos. 7 and 8, in a biological sample involves obtaining a biological sample from a test subject and contacting the biological sample with a compound or an agent capable of detecting said polypeptide or polynucleotide (e.g., mRNA, genomic DNA) such that the presence of said polypeptide or polynucleotide is directly labeled. Examples of indirect labeling include detection of a primary antibody using a fluorescently labeled secondary antibody and end-labeling of a DNA probe with biotin such that it can be detected with fluorescently labeled streptavidin. That is, the detection method of the invention can be used to detect mRNA, protein, or genomic DNA in a biological sample *in vitro* as well as *in vivo.* For example, *in vitro* techniques for detection of mRNA include Northern hybridizations and *in situ* hybridizations. *In vitro* techniques for detection of an angiopoietin-like polypeptide include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of an angiopoietin-like polypeptide include introducing into a subject a labeled antibody directed against the polypeptide.

In one embodiment, the biological sample contains protein molecules from the test subject. Alternatively, the biological sample contains mRNA molecules from the test subject or genomic DNA molecules from the test subject. A preferred biological sample is a peripheral blood sample isolated by conventional means from a subject.

In another embodiment, the methods further involve obtaining a control biological sample from a control subject, contacting the control sample with a compound or agent capable of detecting an angiopoietin-like polypeptide or mRNA or genomic DNA encoding an angiopoietin-like polypeptide, such that the presence of the polypeptide or mRNA or genomic DNA encoding the angiopoietin-like polypeptide is detected in the biological sample, and comparing the presence of said polypeptide or mRNA or genomic DNA encoding an angiopoietin-like polypeptide in the control sample with the presence of the polypeptide or mRNA or genomic DNA encoding the polypeptide in the test sample.

In yet another embodiment, the polynucleotide sequences selected from the group consisting of SEQ ID Nos. 2 and 3, are used as templates for the generation of probes and primers designed for use in identifying and/or cloning angiopoietin-like homologs in other cell types, e.g. from other tissues, as well as angiopoietin-like homologs from other mammalian organisms. Probes and primers of the invention can also be used to determine the identity of an angiopoietin-like allele and/or the presence or absence of one or more mutations in an angiopoietin-like gene of a subject. In a preferred embodiment, a probe or primer of the invention can be used to determine whether a subject has or is at risk of developing a disease or condition associated with a specific angiopoietin-like allele, such as an allele carrying a mutation.

In a preferred embodiment, in the present invention a probe/primer comprising a substantially purified oligonucleotide is used, which oligonucleotide comprises a region of nucleotide sequence that hybridizes under stringent conditions to at least about 12, preferably about 25, more preferably about 40, 50 or 75 consecutive nucleotides of sense or antisense sequence selected from the group consisting of SEQ ID Nos: 2 and 3, or naturally occurring mutants thereof. For instance, primers based on the polynucleotide sequence represented in SEQ ID Nos. 2 and 3 can be used in PCR reactions to clone angiopoietin-like homologs, e.g. specific angiopoietin-like alleles. Such primers are preferably selected in a region that does not share significant homology to other genes, e.g., other angiopoietin genes. Likewise, probes based on angiopoietin-like sequences can be used to detect transcripts or genomic sequences encoding the same or homologous proteins. In preferred embodiments, the probe further comprises a label group attached thereto and able to be detected, e.g. the label group is selected from amongst radioisotopes, fluorescent compounds, enzymes, and enzyme co-factors.

Such probes can also be used as a part of a diagnostic test kit for identifying cells or tissue which mis-express an angiopoietin-like protein, such as by measuring the level of an angiopoietin-like encoding polynucleotide sequence in a sample of cells from a patient; e.g. detecting angiopoietin-like mRNA levels or determining whether a genomic angiopoietin-like gene has been mutated or deleted. Briefly, nucleotide probes can be generated from an angiopoietin-like gene that facilitate histological screening of intact tissue and tissue samples for the presence (or absence) of angiopoietin-like protein encoding transcripts. Similar to the diagnostic uses of anti-angiopoietin-like antibodies, the use of probes directed to angiopoietin-like messages, or to genomic angiopoietin-like sequences, can be used for both predictive and therapeutic evaluation of allelic mutations which might be manifested in, for example, blood disorders (e.g. anemia) (e.g. unwanted cell growth) or abnormal differentiation of tissue. Used in conjunction with immunoassays as described herein, the oligonucleotide probes can help facilitate the determination of the molecular basis for a developmental disorder which may involve some abnormality associated with expression (or lack thereof) of an angiopoietin-like protein. For instance, variation in polypeptide synthesis can be differentiated from a mutation in a coding sequence. Also within the scope of the invention are kits for determining whether a subject is at risk of developing a disease or condition caused by or contributed by an aberrant angiopoietin-like bioactivity and/or which is associated with one or more specific angiopoietin-like alleles. In a preferred embodiment, the kit can be used for determining whether a subject is at risk of developing a blood disorder, e.g. anemia or leukemia.

### Model systems

Cell- and animal-based systems may be used in a variety of applications, for example, to further characterize angiopoietin-like genes and proteins. In addition, such assays may be utilized as part of screening strategies designed to identify compounds that are capable of ameliorating disease symptoms. Thus, the animal- and cell-based models may be used to identify drugs, pharmaceuticals, therapies and interventions that may be effective in treating disease.

### Animal-based systems

One aspect of the present invention concerns transgenic animals comprised of cells (of that animal) which contain a transgene and which preferably (though optionally) express an exogenous angiopoietin-like protein in one or more cells in the animal. Preferably said transgenic animal is a mammal, amphibian, teleost or insect. More preferably said transgenic animal is a mouse, rat or a zebrafish. An angiopoietin-like transgene can encode the wild-type form of the protein, or can encode homologs thereof, including both alleles of angiopoietin-like genes, agonists and antagonists, as well as antisense constructs. In preferred embodiments, the expression of the transgene is restricted to specific subsets of cells, tissues or developmental stages utilizing, for example, cis-acting sequences that control expression in the desired pattern. In the present invention, such mosaic expression of a angiopoietin-like protein can be essential for many forms of lineage analysis and can additionally provide a means to assess the effects of, for example, lack of angiopoietin-like expression which might grossly alter development in small patches of tissue within an otherwise normal embryo. In a preferred embodiment, the invention provides transgenic zebrafish having an allele of the angiopoietin-like gene and the zebrafish can be used, for example, to determine the effect of this specific angiopoietin-like allele on differentiation and/development of specific tissues and cell types (e.g. hematopoietic stem cells, erythroid progenitor cells, vasculature). Tissue-specific regulatory sequences and conditional regulatory sequences can be used to control expression of the transgene in certain spatial patterns. Moreover, temporal patterns of expression can be provided by, for example, conditional recombination systems or prokaryotic transcriptional regulatory sequences. Genetic techniques that allow for the expression of transgenes via site-specific genetic manipulation *in vivo* are known to those skilled in the art. The transgenic animals of the present invention all include within their cells a transgene, which alters the phenotype of the "host cell" with respect to regulation of cell growth, death and/or differentiation. It is noted that those skilled in the art are able to incorporate specific transgene sequences into organisms utilizing any of the methods and materials selected from the group of the cre/loxP recombinase system of bacteriophage P1 (Lakso et al. (1992) PNAS. 89:6232-6236; Orban et al. (1992) PNAS. 89:6861-6865) or the FLP recombinase system of S. cerevisiae (O'Gorman et al. (1991) Science. 251:1351-1355; PCT publication WO 92/15694). Similar conditional transgenes can be provided using prokaryotic promoter sequences that require prokaryotic proteins to be simultaneously expressed in order to facilitate expression of an angiopoietin-like transgene. Exemplary promoters and the corresponding trans-activating prokaryotic proteins are given in U.S. Pat. No. 4,833,080. Furthermore, the transgenic animals of the present invention preferably exhibit alteration of one or more cellular processes of erythroid growth, differentiation, maturation and survival (e.g. conditions resembling anemia or leukemia), alteration in blood vessel development and formation, establishment of stable blood vessel architecture.

Moreover, expression of the conditional transgenes can be induced by gene therapy-like methods wherein a gene encoding the trans-activating protein, e.g. a recombinase or a prokaryotic protein, is delivered to the tissue and caused to be expressed, such as in a cell-type specific manner. By this method, an angiopoietin-like transgene could remain silent into adulthood until "turned on" by the introduction of the trans-activator.

In an exemplary embodiment, the "transgenic non-human animals" of the invention are produced by introducing transgenes into the germ-line of a non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor. For example, when transgenic zebrafish are to be produced, strains such as Tübingen, AB, TL, or Florida wildtype are often used. The line(s) used to practice this invention may themselves be transgenics, and/or may be knockouts (i.e., obtained from animals which have one or more genes partially or completely suppressed).

In one embodiment, the transgene construct is introduced into a single-cell stage zebrafish embryo. The use of zygotes as a target for gene transfer has a major advantage in that in most cases the injected DNA will be incorporated into the host gene before the first cleavage (Brinster et al. (1985) Proc. Natl. Acad. Sci. USA 82:4438-4442). As a consequence, all cells of the transgenic animal will carry the incorporated transgene. This will in general also be reflected in the efficient transmission of the transgene to offspring of the founder since 50% of the germ cells will harbor the transgene.

Introduction of the transgene nucleotide sequence into the zebrafish embryo may be accomplished by any means known in the art, but preferably by microinjection (Meng et al. (1999) Methods Cell. Biol. 60:133-148; Westerfield (2000) The Zebrafish Book). Following introduction of the transgene nucleotide sequence into the embryo, the embryo may be incubated for varying periods of time. Incubation to maturity is within the scope of this invention.

In addition to similar biological considerations, physical ones also govern the amount (e.g., volume) of exogenous genetic material that can be added to the zygote or to the genetic material. The volume of exogenous genetic material inserted will not exceed about 10 nanoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs, which are introduced into the zygote, is dependent upon the total amount of exogenous genetic material added and will be the amount which enables the genetic transformation to occur. Theoretically only one copy is required, however, generally, numerous copies are utilized, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by in vitro fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. The progeny may be evaluated for the presence of the transgene using methods described above, or other appropriate methods.

Retroviral infection can also be used to introduce a transgene into a non-human animal. The developing non-human embryo can be cultured *in vitro* to the blastocyst stage. During this time, the blastomeres can be targets for retroviral infection (Jaenich (1976) PNAS. 73:1260-1264). Alternatively, infection can be performed at a later stage. Virus or virus-producing cells can be injected into the blastocoele (Jahner et al. (1982) Nature. 298:623-628). The viral vector system used to introduce the transgene is typically a replication-defective retrovirus carrying the transgene (Jahner et al. (1985) PNAS. 82:6927-6931; Van der Putten et al. (1985) PNAS. 82:6148-6152). Following the methods and material described previously, those skilled in the art will be able to retrovirally introduce nucleic acids into a non-human embryo to generate transgenic non-human animal.

A third type of target cell for transgene introduction is the embryonal stem cell (ES). ES cells are obtained from pre-implantation embryos cultured in vitro and fused with embryos (Evans et al. (1981) Nature. 292:154-156; Bradley et al. (1984) Nature. 309:255-258; Gossler et al. (1986) Proc.Natl. Acad. Sci. USA 83: 9065-9069; and Robertson et al. (1986) Nature. 322:445-448). Transgenes can be efficiently introduced into the ES cells by DNA transfection or by retrovirus-mediated transduction. For review see Jaenisch (1988) Science. 240:1468-1474.

Gene targeting in embryonic stem cells is in fact a scheme contemplated by the present invention as a means for disrupting an angiopoietin-like gene function through the use of a targeting transgene construct designed to undergo homologous recombination with one or more angiopoietin-like genomic sequences. The targeting construct can be arranged so that, upon recombination with an element of an angiopoietin-like gene, a positive selection marker is inserted into (or replaces) coding sequences of the targeted angiopoietin-like gene. The inserted sequence functionally disrupts the angiopoietin-like gene, while also providing a positive selection trait. Generally, the embryonic stem cells (ES cells) used to produce the knockout animals will be of the same species as the knockout animal to be generated. Thus for example, mouse embryonic stem cells will usually be used for generation of a knockout mouse. Embryonic stem cells are generated and maintained using methods well known to the skilled artisan such as those described by Doetschman et al. ((1985) J. Embryol. Exp. Morphol. 87:27-45). The cells are cultured and prepared for knockout construct insertion using methods well known to the skilled artisan. Insertion of the knockout construct into the ES cells can be accomplished using a variety of methods well known in the art including for example, electroporation, microinjection, and calcium phosphate treatment. A preferred method of insertion is microinjection. For insertion, the knockout construct is added to the ES cells under appropriate conditions for the insertion method chosen, as is known to the skilled artisan. Where more than one construct is to be introduced into the ES cell, each knockout construct can be introduced simultaneously or one at a time.

Screening of the above-described cells can be accomplished using a variety of methods such as antibiotic resistance, Southern blot analysis of ES cell genomic DNA, PCR or enzymatic analysis (for example using specific substrate). One skilled in the art will be familiar with other useful markers and the means for detecting their presence in a given cell. All such markers are contemplated as being included within the scope of the teaching of this invention.

After suitable ES cells containing the knockout construct in the proper location have been identified, the cells can be inserted into an embryo. Insertion may be accomplished in a variety of ways known to the skilled artisan, however a preferred method is by microinjection. For microinjection, about 10-30 cells are collected into a micropipette and injected into embryos that are at the proper stage of development to permit integration of the foreign ES cell containing the knockout construct into the developing embryo. For instance, as the appended Examples describe, the transformed ES cells can be microinjected into blastocytes.

Other means of identifying and characterizing the knockout offspring are available. For example, Northern blots can be used to probe the mRNA for the presence or absence of transcripts encoding either the gene knocked out, the marker gene, or both. In addition, Western blots can be used to assess the level of expression of the angiopoietin-like gene knocked out in various tissues of the offspring by probing the Western blot with an antibody against the particular angiopoietin-like protein, or an antibody against the marker gene product, where this gene is expressed. Finally, *in situ* analysis (such as fixing the cells and labeling with antibody) and/or FACS (fluorescence activated cell sorting) analysis of various cells from the offspring can be conducted using suitable antibodies to look for the presence or absence of the knockout construct gene product.

Yet other methods of making knockout or disruption transgenic animals are also generally known. (See, e.g. Koster et al. (2001) Dev. Biol. 233:329-346; Huang et al. (2001) Mol. Cell. Endocrinol. 177:117-124; Long et al. (1997) Development 124:4105-4111; Motoike et al. (2000) Genesis 28:75-81.

Recombinase dependent knockouts can also be generated, e.g. by homologous recombination to insert target sequences, such that tissue specific and/or temporal control of inactivation of an ANGPTL-gene can be controlled by recombinase sequences.

Animals containing more than one knockout construct and/or more than one transgene expression construct are prepared in any of several ways. The preferred manner of preparation is to generate a series of mammals, each containing one of the desired transgenic phenotypes. Such animals are bred together through a series of crosses, backcrosses and selections, to ultimately generate a single animal containing all desired knockout constructs and/or expression constructs, where the animal is otherwise congenic (genetically identical) to the wild type except for the presence of the knockout construct(s) and/or transgene(s).

The transgenic animals produced in accordance with the present invention will include exogenous genetic material. As set out above, the exogenous genetic material will, in certain embodiments, be a DNA sequence that results in the production of an angiopoietin-like protein (either agonistic or antagonistic), an antisense transcript, or an angiopoietin-like mutant. Further, in such embodiments the sequence will be attached to a transcriptional control element, e.g., a promoter, which preferably allows the expression of the transgene product in a specific type of cell.

### Antisense oligomers

A further aspect of the invention relates to "antisense" therapy. As used herein, "antisense" therapy refers to the administration or *in situ* generation of oligomer molecules or their derivatives which specifically hybridize (e.g. bind) under cellular conditions, with the cellular mRNA and/or genomic DNA encoding one or more angiopoietin-like proteins so as to inhibit expression of that protein(s), e.g. by inhibiting transcription and/or translation. The binding may be by classical base pair complementarity, or, for example, in the case of binding to DNA duplexes, through specific interactions in the major groove of the double helix. In general, "antisense" therapy refers to the range of techniques generally employed in the art, and includes any therapy that relies on specific binding of oligomers to target polynucleotide sequences.

An antisense construct of the present invention can be delivered, for example, as an expression plasmid which, when transcribed in the cell, produces RNA which is complementary to at least a unique portion of the cellular mRNA which encodes an angiopoietin-like protein. Alternatively, the antisense construct is an oligomer probe that is generated *ex vivo* and which, when introduced into the cell causes inhibition of expression by hybridizing with the mRNA and/or genomic sequences of an angiopoietin-like gene. Such oligomer probes are preferably modified oligomers that are resistant to endogenous nucleases, e.g. exonucleases and/or endonucleases, and are therefore stable *in vivo.* Molecules for use as antisense oligomers are selected from the group of, but not limited to phosphodiamidate, phosphoramidate, phosphorothioate and methylphosphonate analogs of DNA (Froehler et al. (1988) Nucleic Acid Res. 16:4831-4839; Hyrup and Nielsen (1996) Bioorg. Med. Chem. 4:5-23, Sarin et al. (1988) Proc. Natl. Acad. Sci. USA. 85:7448-7451; Stein et al. (1988) Nucleic Acid Res. 16:3209-21; Summerton (1999) Biochim. Biophys. Acta. 1489:141-158; Summerton and Weller (1997) Antisense Nucleic Acid Drug Dev. 7:187-195; Orum and Wengel (2001); Wahlestedt et al., (2000). Additionally, general approaches to constructing oligomers useful in "antisense" therapy have been reviewed, in Letsinger et al. (1989) Proc. Natl. Acad. Sci. USA. 86:6553-6556; Summerton and Weller (1997) Antisense Nucleic Acid Drug Dev. 7:187-195; Froehler et al. (1988) Nucleic Acid Res. 16:4831-4839. With respect to antisense nucleotide sequences, oligomers derived from the translation initiation site, e.g., between the -10 and +10 regions of the angiopoietin-like nucleotide sequence of interest, are preferred.

Antisense approaches involve the design of oligonucleotides (either DNA or RNA, analogs, or derivatives thereof) that are complementary to angiopoietin-like mRNA. The antisense oligomers will bind to the angiopoietin-like mRNA transcripts and prevent translation. Absolute complementarity, although preferred, is not required. A sequence "complementary" to a portion of an RNA, as referred to herein, means a sequence having sufficient complementarity to be able to hybridize with the RNA, forming a stable duplex; in the case of double-stranded antisense polynucleotide sequences, a single strand of the duplex DNA may thus be tested, or triplex formation may be assayed. The ability to hybridize will depend on both the degree of complementarity and the length of the antisense polynucleotide sequence. Generally, the longer the hybridizing polynucleotide sequence, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligomers that are complementary to the 5' end of the message, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner (1994) Nature. 372:333-335). Therefore, oligomers complementary to either the 5' or 3' untranslated, non-coding regions of an angiopoietin-like gene could be used in an antisense approach to inhibit translation of endogenous angiopoietin-like mRNA. Oligomers complementary to the 5' untranslated region of the mRNA should include the complement of the AUG start codon. Antisense oligomers complementary to mRNA coding regions are less efficient inhibitors of translation but could be used in accordance with the invention. Whether designed to hybridize to the 5', 3' or coding region of angiopoietin-like mRNA, antisense oligomers should be at least six nucleotides in length, and are preferably oligomers ranging from 6 to about 50 nucleotides in length. In certain embodiments, the oligomer is at least 10 nucleotides, at least 17 nucleotides, at least 25 nucleotides, or at least 50 nucleotides in length.

Regardless of the choice of the target sequence, it is preferred that *in vitro* studies are first performed to quantitate the ability of the antisense oligomer to inhibit gene expression. It is preferred that these studies utilize controls that distinguish between antisense gene inhibition and nonspecific biological effects of oligomers. It is also preferred that these studies compare levels of the target RNA or protein with that of an internal control RNA or protein. Additionally, it is envisioned that results obtained using the antisense oligomer are compared with those obtained using control oligomers. It is preferred that the control oligonucleotide is of approximately the same length as the test oligomers and that the nucleotide sequence of the oligomers differs from the antisense sequence no more than is necessary to prevent specific hybridization to the target sequence.

The oligomers can be DNA or RNA or chimeric mixtures or derivatives or modified versions thereof, single-stranded or double-stranded. The oligomer can be modified at the base moiety, sugar moiety, or phosphate backbone, for example, to improve stability of the molecule, hybridization, etc. The oligomer may include other appended groups such as peptides (e.g. for targeting host cell receptors *in vivo),* or agents facilitating transport across the cell membrane (see Letsinger et al. (1989) Proc. Natl. Acad. Sci. USA. 86:6553-6556; Lemaitre et al. (1987) Proc. Natl. Acad. Sci. USA. 84:648-652; PCT Publication No. WO 88/09810, published Dec. 15, 1988) or the blood-brain barrier (see PCT Publication No. WO 89/10134, published Apr. 25, 1988), hybridization-triggered cleavage agents. (Van der Krol et al. (1988) Biotechniques 6:958-976) or intercalating agents (Zon (1988) Pharm. Res. 5:539-549). To this end, the oligomer may be conjugated to another molecule, e.g., a peptide, hybridization triggered cross-linking agent, transport agent, hybridization-triggered cleavage agent, etc.

The antisense oligomer may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5oxyacetic acid methylester, uracil-5-oxyacetic acid (v), -5-methyl-2-thiouracil, 3-(3-amino-3-N-2-carboxypropyl) uracil, (acp3)w, and 2,6-diaminopurine.

The antisense oligomer may also comprise at least one modified sugar moiety selected from the group including but not limited to arabinose, 2-fluoroarabinose, xylulose, and hexose. In yet another embodiment, the antisense oligomer comprises at least one modified phosphate backbone selected from the group consisting of a phosphorothioate (Stein and Cohen (1989) In: Oligonucelotides: Antisense Inhibitors of Gene Expression. pp.97-117), a phosphorothioate, a phosphoramidothioate, a phosphoramidate (Froehler et al. (1988), Nucleic Acid. Res. 16:4831-4839), a phosphordiamidate, a methylphosphonate (Miller (1989) In: Oligonucelotides: Antisense Inhibitors of Gene Expression, pp.85-92), an alkyl phosphotriester (Miller (1989) In: Oligonucelotides: Antisense Inhibitors of Gene Expression. pp.82-85), a formacetal or analog thereof, as well as α-DNA (Rayner et al. (1989) In: Oligonucelotides: Antisense Inhibitors of Gene Expression. pp.119-136), 2'-O-methyl RNA (Shibahara et al. (1989), Nucleic Acid Res. 17:239-252), and morpholine backbone (Summerton and Weller (1997) Antisense Nucleic Acid Drug Dev. 7:187-195). Oligomers of the invention may be synthesized by standard methods known in the art, e.g. by use of an automated DNA synthesizer (such as are commercially available from Biosearch, Applied Biosystems, etc.). As examples, phosphorothioate oligomers may be synthesized by the method of Stein et al. (1988) Nucleic Acid Res. 16:3209-3021), methylphosphonate oligomers can be prepared by use of controlled pore glass polymer supports (Sarin et al. (1988) Proc. Natl. Acad. Sci. USA. 85:7448-7451). Morpholino oligomers may be synthesised by the method of Summerton and Weller (1993) U.S. Patent Nos. 5,217,866 and 5,185,444, etc.

In specific embodiments, antisense molecules are delivered to cells that express angiopoietin-like proteins *in vivo.* A number of methods have been developed for delivering antisense DNA or RNA to cells; e.g., antisense molecules can be injected directly into the tissue site, or modified antisense molecules, designed to target the desired cells e.g., antisense linked to peptides or antibodies that specifically bind receptors or antigens expressed on the target cell surface can be administered systematically.

### Polypeptides used in the present invention

One aspect of the invention pertains to the use of polypeptide sequences selected from the group coonsisting of SEQ ID Nos. 7 or 8 and equivalents thereof for the treatment of blood related disorders or defects in vasculature. The term equivalent is understood to include functionally equivalent polypeptides having an activity of an angiopoietin-like protein such as described herein.

Polypeptides, used according to the present invention, are at least 55% identical or similar to the amino acid sequences of SEQ ID Nos. 7 or 8. Polypeptides which are at least about 70%, and even more preferably at least about 80%, 85%, 90%, 95%, or 98% identical or similar to the amino acid sequences represented in SEQ ID Nos. 7 or 8 are also within the scope of the invention.

In some preferred embodiments of the invention polypeptides are used which are at least about 70%, preferably at least about 80%, and most preferably at least about 90% identical orsimilar to a portion of at least about 10 consecutive amino acids of the amino acid sequence set forth in SEQ ID Nos. 7 or 8. In one embodiment, the portion corresponds to about amino acid 1 to about amino acid 493 of SEQ ID Nos. 7 or 8.

In a particularly preferred embodiment, polypeptides have an overall amino acid sequence homology or identity of at least about 70%, at least about 80%, at least about 85%, at least about 90%, at least about 95%, at least about 98%, or at least about 99% with the amino acid sequences shown in SEQ ID Nos. 7 or 8 are used according to the present invention. The polypeptides include angiopoietin-like proteins from any species. Preferred polypeptides are vertebrate angiopoietin-like proteins. More preferred polypeptides are those of primate angiopoietin-like proteins including mammalian angiopoietin-like proteins, e.g., human angiopoietin-like proteins. Other polypeptides used in the invention include fish, amphibian, avian, equine, canine, feline, bovine or porcine angiopoietin-like proteins.

In a preferred embodiment, the polypeptide includes a polypeptide sequence corresponding to all or a portion of the amino acid sequence of SEQ ID Nos. 7 or 8, e.g. at least 2, 5, 10, 25, 50, 100, 150 or 200 amino acids of that region. Yet other preferred polypeptides comprise at least about 3, at least about 5, at least about 10, at least about 15, at least about 20, or at least about 25 consecutive amino acids from about amino acid 1 to about amino acid 493 set forth in SEQ ID Nos. 7 or 8.

In another preferred embodiment of the invention, the polypeptides comprise a signal sequence, coiled-coil domain and a fibrinogen-like domain, such as, angiopoietin-like proteins having SEQ ID Nos. 7 or 8. Other preferred polypeptides comprise a fibrinogen-like domain. Yet other preferred polypeptides comprise at least of one of the conserved motifs in the fibrinogen domain, e.g. Ca⁺⁺-binding motif. In another preferred embodiment, the polypeptides correspond to angiopoietin-like proteins essentially lacking the fibrinogen-like domain. Other preferred polypeptides comprise a conserved coiled-coil domain of angiopoietins.

Polypeptides selected from the above-described amino acid sequences, can be soluble. Preferred soluble peptides comprise at least a portion of the fibrinogen-like domain of an angiopoietin-like protein. Even more preferred, soluble polypeptides comprise a portion of the fibrinogen-like domain, including the Ca⁺⁺-binding motif of an angiopoietin-like protein consisting of the amino acid sequence SEQ ID No. 9. Furthermore, soluble polypeptides that comprise a portion of the fibrinogen-like domain, the Ca⁺⁺-binding motif, and the fibrinogen beta and gamma chains in the C-terminal domain consisting of the amino acid SEQ ID No. 10 are envisioned by the present invention. Moreover, such preferred soluble polypeptides may comprise a portion of the fibrinogen-like domain, the Ca⁺⁺-binding motif, the fibrinogen beta and gamma chains in the C-terminal domain and a coiled-coil domain. Such soluble polypeptides may in addition comprise a signal sequence consisting of SEQ ID No. 11.

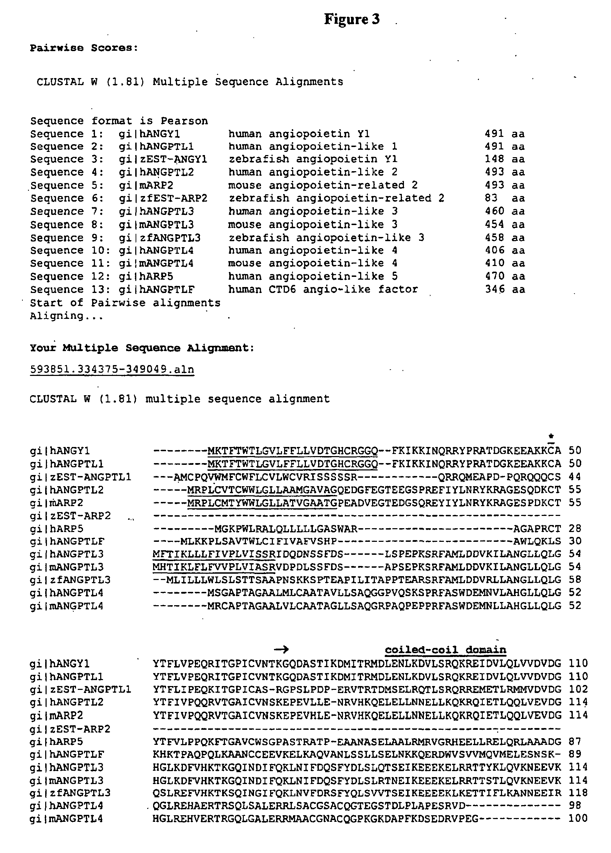

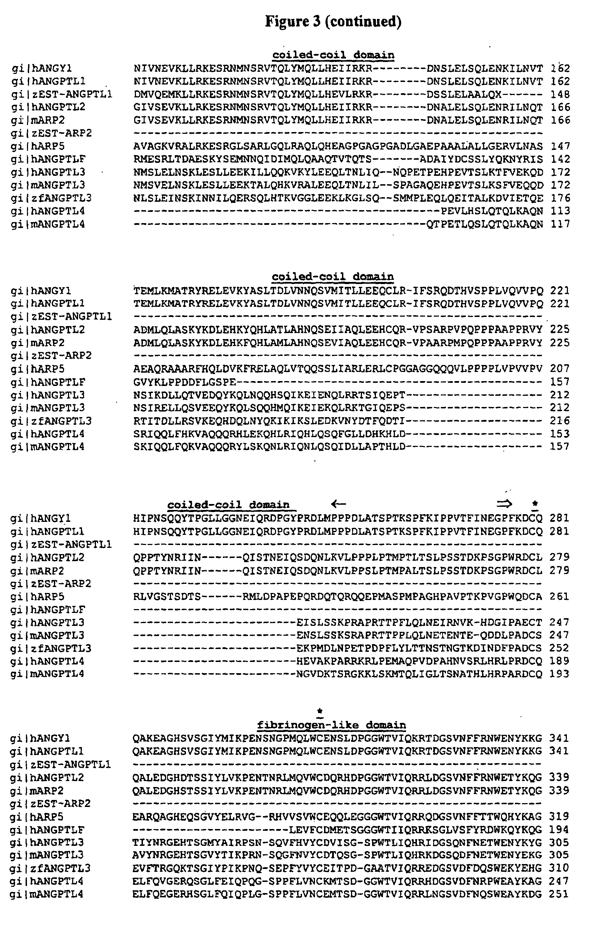

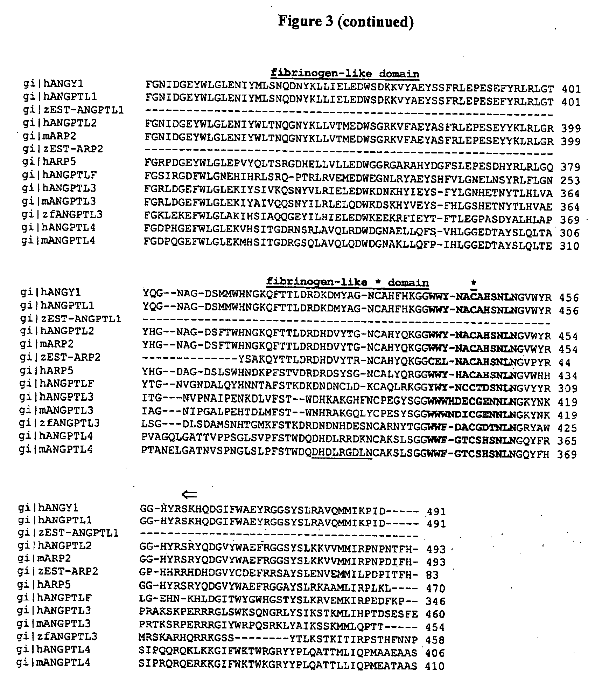

In yet other preferred embodiments, the polypeptides used in the invention comprise at least one domain of an angiopoietin-like protein selected from the group consisting of: a fibrinogen-like domain, an Ca⁺⁺-binding motif consisting of the amino acid sequence SEQ ID No. 9, a fibrinogen beta and gamma chains in the C-terminal domain consisting of the amino acid sequence of SEQ ID No.10, a coiled-coil domain, and/or a signal sequence consisting of the amino acid sequence of SEQ ID No.11, and all phosphorylation sites required for the polypeptides to be activated (Figure 3). The polypeptides can comprise several of these domains from an angiopoietin-like protein. Alternatively, a polypeptide can be a chimeric protein, i.e., comprising several of these conserved domains, at least some of which are derived from a protein other than an angiopoietin-like protein.

Polypeptides and fragment polypeptides that are capable of exhibiting biological activity are also used in the present invention. Fragments of the protein may be in linear form or they may be cyclized using known methods, for example, as described in Saragovi et al. (1992) Biotechnology. 10:773-778.

Angiopoietin-like polypeptides as fusion proteins can be used in this invention. Such fusion proteins can comprise at least a portion of the kinase domain of an angiopoietin-like polypeptide. Polynucleotide sequences encoding such fusion proteins can be prepared as described in U.S. Patent No. 5,434,131.

### Polynucleotides encoding polypeptides used in the present invention

Another aspect of the invention pertains to the use of isolated polynucleotide sequences comprising nucleotide sequences encoding angiopoietin-like polypeptides, and/or equivalents thereof. The term equivalent is understood to include nucleotide sequences encoding functionally equivalent angiopoietin-like polypeptides or functionally equivalent peptides having an activity of an angiopoietin-like protein such as described herein. Equivalent nucleotide sequences will include sequences that differ by one or more nucleotide substitutions, additions or deletions, such as allelic variants; and will, therefore, include sequences that differ from the polynucleotide sequences of the angiopoietin-like genes shown in any of SEQ ID Nos. 1, 2, 3, or 4 due to the degeneracy of the genetic code.

Polynucleotides encoding polypeptide sequences set forth in theforegoing section "Polypeptides used in the present invention" are all envisioned for use in the various embodiments of the present invention.

Preferred polynucleotides have a sequence at least about 75% homologous and more preferably at least about 80% and even more preferably at least about 85% homologous with a polynucleotide sequence of an angiopoietin-like gene, such as human angiopoietin-like 1 gene, e.g., such as a sequence shown in one of SEQ ID Nos. 2 or 3. Polynucleotide sequences at least about 90%, more preferably at least about 95%, and most preferably at least about 98-99% homologous with a polynucleotide sequence represented in one of SEQ ID Nos. 2 or 3 are of course also within the scope of the invention. In preferred embodiments, the polynucleotide is a human angiopoietin-like gene and, in particularly preferred embodiments, includes all or a portion of the nucleotide sequence corresponding to the coding region of one of SEQ ID Nos. 2 or 3.

Furthermore, polynucleotide sequences having sequences that differ from the polynucleotide sequences shown in one of SEQ ID Nos. 2 or 3 due to degeneracy of the genetic code, can be used in the invention. Such polynucleotides encode functionally equivalent peptides (i.e., a peptide having a biological activity of a angiopoietin-like polypeptide) but differ in sequence from the sequence shown in the sequence listing due to degeneracy in the genetic code. For example, a number of amino acids are designated by more than one triplet. Codons that specify the same amino acid, or synonyms (e.g., GCU, GCC, GCA, and GCG encode alanine), may result in "silent" mutations that do not affect the amino acid sequence of an angiopoietin-like polypeptide. However, it is expected that DNA sequence polymorphisms that do lead to changes in the amino acid sequences of the subject angiopoietin-like polypeptides will exist. One skilled in the art will appreciate that these variations in one or more nucleotides (e.g. up to about 3-5% of the nucleotides) of the polynucleotide encoding polypeptides having a biological activity of an angiopoietin-like polypeptide may exist among individuals of a given species due to natural allelic variation.

Angiopoietin-like protein encoding polynucleotide sequences can be obtained from mRNA present in any of a number of eukaryotic cells. It should also be possible to obtain polynucleotide sequences encoding angiopoietin-like polypeptides used in the present invention from genomic DNA from both adults and embryos. For example, screening cDNA or genomic libraries (Benton et al. (1977) Science. 196:180-182). Purification of such polynucleotides from suitable cell lines or libraries, specific for a particular tissue (e.g. ATCC No. CCL-246, a cell line derived from human bone marrow, specific for myelogenous leukemia). A cDNA encoding an angiopoietin-like protein can be obtained by isolating total mRNA from a cell, e.g. a vertebrate cell, a mammalian cell, or a human cell, including embryonic cells, in accordance with protocols described within Current Protocols in Molecular Biology (1989) 4.5.1-4.5.3. The gene encoding an angiopoietin-like protein can also be cloned using established polymerase chain reaction techniques (PCR) in accordance with the nucleotide sequence information provided by the invention (Saiki et al. (1988) Science. 239:487-491; and Schaefer (1995) Analytical Biochem. 227:255-273). The polynucleotide sequence of the invention can be DNA or RNA. A preferred polynucleotide sequence is a cDNA represented by a sequence selected from the group consisting of SEQ ID Nos. 2 or 3.

### Probes and Primers

Moreover, the polynucleotide sequences determined from human angiopoietin-like genes will further allow for the generation of probes and primers designed for use in identifying and/or cloning angiopoietin-like homologs in other cell types, e.g. from other tissues, as well as angiopoietin-like homologs from other mammalian organisms. Probes and primers can also be used to determine the identity of an angiopoietin-like allele and/or the presence or absence of one or more mutations in an angiopoietin-like gene of a subject. In a preferred embodiment, a probe or primer used in the invention can be used to determine whether a subject has or is at risk of developing a disease or condition associated with a specific angiopoietin-like allele, such as an allele carrying a mutation.

Preferred polynucleotide sequences for use as a probe, according to the methods of the invention, include polynucleotide sequences comprising a nucleotide sequence having at least about 6, preferably at least about 9, more preferably at least about 12 and even more preferably at least about 15 consecutive nucleotides from SEQ ID Nos. 2 or 3 or from a portion thereof. In a preferred embodiment, the portion corresponds to about nucleotide 1 to about nucleotide 2066 of SEQ ID Nos. 2 or 3. Alternatively a portion can be a nucleotide sequence encoding a conserved motif of human angiopoietin-like protein, e.g., coiled-coil or fibrinogen-like domain. Alternatively, the portion can be a nucleotide sequence located between polynucleotide sequences encoding conserved motifs of human angiopoietin-like protein.

The invention further pertains to polynucleotide sequence molecules for use as probes/primer or antisense molecules (i.e. non-coding polynucleotide sequence molecules), which can comprise at least about 6, 12, 20, 30, 50, 100, 125, 150 or 200 nucleotides or base pairs. Yet other preferred polynucleotide sequences comprise at least about 300, at least about 350, at least about 400, at least about 450, at least about 500, or at least about 600 nucleotides of SEQ ID Nos. 2 or 3. In some embodiments, the polynucleotide sequences of the invention correspond to the 5' portion of the polynucleotide sequences shown SEQ ID Nos. 2 or 3. For example, a polynucleotide sequence used in the invention can correspond to a portion of about nucleotide 1 to about nucleotide 2066 of the polynucleotide sequences shown in SEQ ID Nos. 2 or 3.

Also used in the invention are polynucleotide sequences that are capable of hybridizing to an angiopoietin-like polypeptide sequence, and in particular, to those shown in SEQ ID Nos. 2 or 3, and fragments thereof under various conditions of stringency. Conditions that promote hybridization of polynucleotide sequences are known to those skilled in the art (see Current Protocols in Molecular Biology, John Wiley & Sons, N.Y. (1989) 6.3.1-6.3.6, Jowett (2001) Methods. 23:345-358). For example, stringent salt concentration will ordinarily be less than about 750mM NaCI and 75mM trisodium citrate. High stringency hybridization can be obtained in the presence of at least about 50% formamide. Stringent temperature conditions will ordinarily include temperatures of at least 45 degree Celsius, more preferably of at least 55 degree Celsius, and most preferably of at least about 65 degree Celsius. Varying additional parameters, such as hybridization time, the concentration of detergent, e.g., Tween 20, and the inclusion or exclusion of carrier DNA, are well known to those skilled in the art. Various levels of stringency can be accomplished by combining these various conditions as needed. In a preferred embodiment, hybridization will occur at 45 degree Celsius in 750mM NaCl, 75mM trisodium citrate, and 0.1% Tween 20, 50% formamide, 500µg/ml yeast RNA and 50µg/ml heparin. In a more preferred embodiment hybridization will occur at 55 degree Celsius in 750mM NaCI, 75mM trisodium citrate, and 0.1% Tween 20, 50% formamide, 500µg/ml yeast RNA and 50µg/ml heparin. In a more preferred embodiment hybridization will occur at 65 degree Celsius in 750mM NaCI, 75mM trisodium citrate, and 0.1% Tween 20, 50% formamide, 500µg/ml yeast RNA and 50µg/ml heparin. Useful variation on these conditions will be readily apparent to those skilled in the art. The stringency of the wash steps, which follow hybridization, is defined by the salt concentration and by temperature. For example, stringent salt concentrations for the wash steps will preferably be less than about 30mM NaCl and 3mM trisodium citrate. Stringent temperature conditions for the wash steps will ordinarily include temperatures of at least 45 degree Celsius, more preferably at least 55 degree Celsius, and most preferably at 65 degree Celsius. In a preferred embodiment, wash steps will occur at 65 degree Celsius in 30mM NaCl, 3mM trisodium citrate, and 0.1% Tween-20. Additional variation on these conditions will be readily apparent to those skilled in the art.

In a preferred embodiment, the present invention also provides a probe/primer comprising a substantially purified oligonucleotide, which oligonucleotide comprises a region of nucleotide sequence that hybridizes under stringent conditions to at least about 12 (e.g. 12), preferably about 25 (e.g. 25), more preferably about 40, 50 or 75 (e.g. 40, 50, or 75 respectively) consecutive nucleotides of sense or antisense sequence of the nucleotide sequence selected from the group consisting of SEQ ID Nos. 2 or 3, or naturally occurring mutants thereof. For instance, primers based on the polynucleotide sequence represented in SEQ ID Nos. 2 or 3 can be used in PCR reactions to clone angiopoietin-like homologs, e.g. specific angiopoietin-like alleles. Such primers are preferably selected in a region that does not share significant homology to other genes, e.g., other fibrinogen domain containing genes. Likewise, probes based on the subject angiopoietin-like sequences can be used to detect transcripts or genomic sequences encoding the same or homologous proteins. In preferred embodiments, the probe further comprises a label group attached thereto and able to be detected, e.g. the label group is selected from amongst radioisotopes, fluorescent compounds, enzymes, and enzyme co-factors.

Such probes can also be used as a part of a diagnostic test kit for identifying cells or tissue which mis-express an angiopoietin-like protein, such as by measuring a level of an angiopoietin-like protein encoding polynucleotide sequence in a sample of cells from a patient; e.g. detecting angiopoietin-like mRNA levels or determining whether a genomic angiopoietin-like gene has been mutated or deleted. Briefly, nucleotide probes can be generated from the subject angiopoietin-like genes that facilitate histological screening of intact tissue and tissue samples for the presence (or absence) of angiopoietin-like protein encoding transcripts. Similar to the diagnostic uses of anti-angiopoietin-like antibodies, the use of probes directed to angiopoietin-like messages, or to genomic angiopoietin-like sequences, can be used for both predictive and therapeutic evaluation of allelic mutations which might be manifested in, for example, blood disorders (e.g. anemia) (e.g. unwanted cell growth) or abnormal differentiation of tissue. Used in conjunction with immunoassays as described herein, the oligonucleotide probes can help facilitate the determination of the molecular basis for a developmental disorder which may involve some abnormality associated with expression (or lack thereof) of an angiopoietin-like protein. For instance, variation in polypeptide synthesis can be differentiated from a mutation in a coding sequence. Also within the scope of the invention are kits for determining whether a subject is at risk of developing a disease or condition caused, or contributed, by an aberrant angiopoietin-like bioactivity and/or which is associated with one or more specific angiopoietin-like alleles. In a preferred embodiment, the kit can be used for determining whether a subject is at risk of developing a blood disorder, e.g. anemia or leukemia, or defects in vasculature.

### Vectors

Expression vectors containing a polynucleotide sequences encoding an angiopoietin-like polypeptide and being operably linked to at least one transcriptional regulatory sequence may be used for practicing the present invention. Regulatory sequences are recognized by those skilled in the art, and are specifically selected to direct expression of the subject angiopoietin-like proteins. Such regulatory sequences are described in Rodriguez and Chamberlain (1982) eds. In: Promoters: Structure and function. NY. Praeger; Khoury and Gruss (1983) Cell. 33:313-314. In one embodiment, the expression vector includes a recombinant gene encoding a peptide having an agonistic activity of a subject angiopoietin-like polypeptide, or alternatively, encoding a peptide which is an antagonistic form of the angiopoietin-like protein. Such expression vectors can be used to transfect cells and thereby produce polypeptides, including fusion proteins, encoded by polynucleotide sequences as described herein. Moreover, the gene constructs can be used as a part of a gene therapy protocol to deliver polynucleotide sequences encoding either an agonistic or antagonistic form of an angiopoietin-like protein. Thus, another aspect of the invention features expression vectors for *in vivo, in vitro,* or *ex vivo* transfection and expression of an angiopoietin-like polypeptide in particular cell types so as to reconstitute the function of, or alternatively, abrogate the function of angiopoietin-like activity (e.g. stimulation of vascular remodeling or activating differentiation of hematopoietic progenitor cells into blood cells). This could be desirable, for example, when the naturally occurring form of the protein is mis-expressed; or to deliver a form of the protein which alters differentiation of tissue (e.g. inhibiting or altering differentiation of hematopoietic stem cells).

In addition to viral transfer methods, non-viral methods can also be employed to cause expression of angiopoietin-like polypeptides in the tissue of an animal. Most nonviral methods of gene transfer rely on normal mechanisms used by mammalian cells for the uptake and intracellular transport of macromolecules. In preferred embodiments, non-viral targeting means rely on endocytic pathways for the uptake of an angiopoietin-like polypeptide gene by the targeted cell. Exemplary targeting means of this type include liposomal derived systems, poly-lysine conjugates, and artificial viral envelopes.

The present invention is further illustrated by the following examples, which should not be construed as limiting in any way. The contents of all cited references (including literature references, issued patents, published patent applications), as cited throughout this application, are hereby expressly incorporated by reference. The practice of the present invention will employ, unless otherwise indicated, conventional techniques of cell biology, cell culture, molecular biology, transgenic biology, microbiology, recombinant DNA, and immunology, which are known to those skilled in the art. Such techniques are explained fully in the literature.

### Example 1. Homology analyses of Angiopoietin Y1

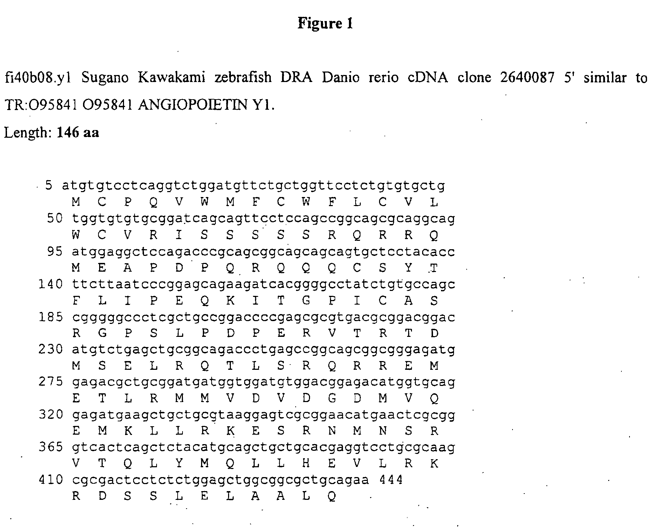

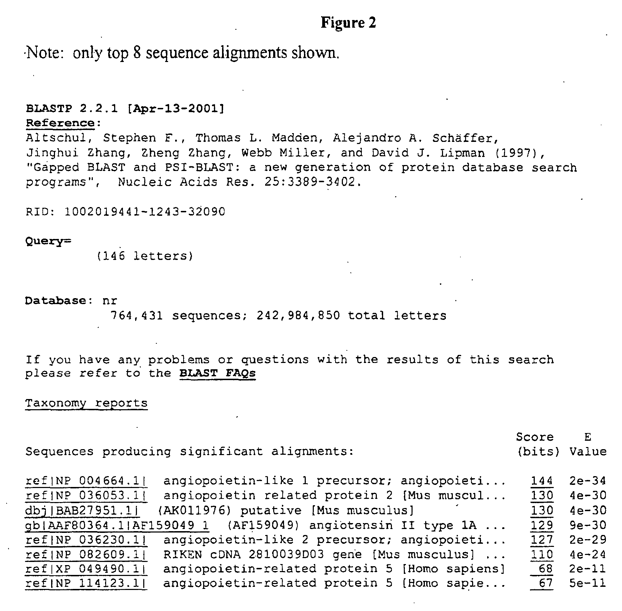

A search for angiopoietin family members in the zebrafish expressed sequence tag (zfEST) database of the National Center for Biotechnology Information (NCBI) revealed the existence of a partial cDNA with Accession Number AW174181 (Sugano et al., 1999). The zfEST was analyzed using the NCBI ORF Finder program in order to identify the longest open reading frame of the partial cDNA. The resulting nucleotide sequence was then translated into a polypeptide sequence (ORF Finder program) and a BLASTP search (Altschul et al., 1997) to identify homologous protein sequences was performed. Results of the BLASTP analysis showed that the gene encoded a polypeptide possessing high homology to the angiopoietin-like (ANGPTL) family of proteins (see Figures 1 and 2). Alignment of the zebrafish translated-EST for angiopoietin Y1 (ANGY1) with the amino acid sequences for human and mouse ANGPTL1 (ANGY1), ANGPTL2 (or angiopoietin-related; ARP2), ANGPTL3, ANGPTL4, ANGPTL5 (ARP5) and ANGPTLF (angiopoietin-like factor) reveals that the isolated zebrafish EST is closely related to a subset of the ANGPTL family, mainly ANGPTL1 and ANPTL2 (based on results of a ClustalW computational analysis). In particular, the isolated zebrafish EST holds the highest degree of homology to human ANGPTL1, indicating that this partial nucleotide sequence is most likely the zebrafish homologue. Furthermore, alignment of amino acid sequences for angiopoietin-like proteins in human, mouse and zebrafish also revealed striking sequence similarities across species, particularly within several functional domains. Both human and mouse ANGPTL1 and ANGPTL2 contain a secretory signal sequence (SEQ ID NO: 11) at the N-terminus (Figure 3, underlined), which is also conserved in zebrafish. An N-terminal coiled-coil domain (Figure 3, marked by small solid arrows), similar to the coiled-coil domains found in most angiopoietins (ANG), encompasses one third of the polypeptide. Coiled-coil domains of angiopoietins have been shown to mediate oligomerization of angiopoietin molecules, *in vitro* (Propocio et al., 1999). Based on amino acid alignments of ANG1, ANG2, ANGPTL1 and ANGPTL2, this coiled-coil domain is well conserved within the angiopoietin family, suggesting a similar role in ANGPTL molecules (Propocio et al., 1999). This coiled-coil is followed by a short sequence of amino acids that links this domain with that of the fibrinogen-like domain; hence linker (Conklin et al., 1999). The fibrinogen-like domain, (Figure 3, marked by large, outlined arrows) is not only highly conserved across species, but also across the angiopoietin family of proteins as the receptor-ligand binding domain where it functions as a mediator of agonistic (ANG1) and antagonistic (ANG2) effects of these ligand (Xu et al., 1996; Propocio et al., 1999). Furthermore, this domain contains a Ca⁺⁺-binding motif (SEQ ID NO: 9) (Figure 3, underlined) also present in most members of the angiopoietin-like family of proteins (Conklin et al., 1999), as well as a signature 'fibrinogen beta and gamma chains' domain at the C-terminal end of the molecule (SEQ ID NO: 10) (Figure 3, bold). Moreover, several conserved cysteine residues can be found within the aligned angiopoietin-like proteins (Figure 3, underlined astericks). These cysteine residues form disulfide bridges similar to those found in angiopoietin molecules, indicating that the secondary structure of angiopoietin-like molecules is comparable to that of the angiopoitein molecules. Interestingly, ANGPTL1 and ANGPTL2 contain an extra cystein residue within the N-terminal portion of the protein which is not found in other members of the angiopoietin-like family of proteins. This amino acid residue is conserved in angiopoietin 1 (ANG1) and angiopoietin 2 (ANG2) (Kim et al., 1999b).

### Example 2. Inhibition of the zebrafish angiopoietin-like 1 precursor (zfANGPTL1) transcript via morpholino antisense mediated knockdown

In order to determine whether ANGTPL1 plays a role in hematopoiesis/erythropoiesis, an antisense oligomer of the morpholino structural type was designed to target the region flanking the initiation codon of the zebrafish ANGPTL1 transcript (nucleotides 2-26 of zfEST, with ATG starting at nucleotide 5), resulting in inhibition of protein synthesis (gene 'knock-down'). The said zfANGPTL1-specific morpholino oligomer was synthesized with the following nucleotide base sequence:

5'-ACATCCAGACCTGAGGACACATAGC-3' (SEQ ID NO: 5).

Approximately 0.25 nl to 1.0 nl of said morpholino oligomer at 1:10 and 1:50 dilutions of a 2mM stock solution, were microinjected into single-cell stage zebrafish embryos using back-filled fine borosilicate glass capillary needles (Ekker, 2000; Nasevicius and Ekker, 2000). Live morphological analysis of treated embryos at 1 day post-fertilization (dpf) revealed no obvious morphological defects.

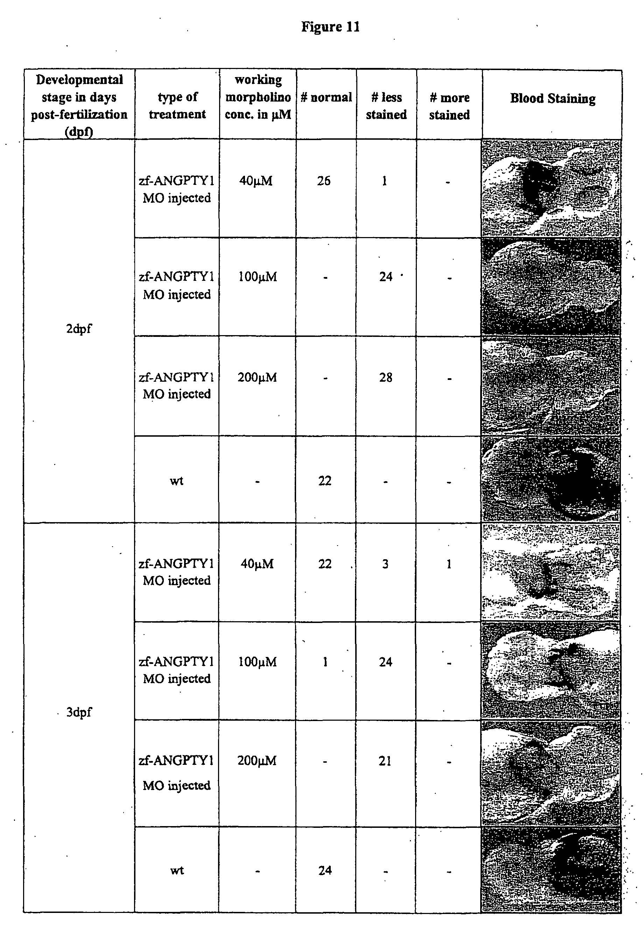

Since hematopoiesis in the zebrafish begins before 1 dpf, o-dianisidine staining of hemoglobin was performed with embryos injected with 40µM, 100µM and 200µM of said morpholino oligomer, after 2dpf and 3dpf. O-dianisidine staining (Detrich III, et al., 1995) of same treated zebrafish embryos at 2 dpf revealed a dose-dependent effect of said morpholino oligomer on hemoglobin staining (Figure 11). At 40µM, the overall hemoglobin staining is unaffected and all embryos appear normal. However, at both 100 and 200µM, there is a complete loss of hemoglobin staining, suggesting that zfANGPTL1 plays a role in either hematopoiesis or erythropoiesis. Wild-type (wt) embryos embryos showed no signs of alterations in hemoglobin staining, indicating that the loss of hemoglobin staining in embryos injected with said morpholino oligomer was specific to the gene 'knock-down' of zfANGPTL1 (Figure 11). Control embryos injected with a morpholino oligomer designed specifically against uroporphyrinogen decarboxylase (UROD) revealed expected results (data not shown). Inhibition of protein synthesis of UROD, a decarboxylase important in the heme synthesis pathway, results in the inability of the erythrocyte/reticulocyte to convert uroporphyrinogen→coproporphyrinogen, thereby causing an accumulation of uroporphyrinogen within the cell cytoplasm, and ultimately to an absence of heme.

Interestingly, o-dianisidine staining analysis of treated embryos after 3dpf revealed similar results (Figure 11). Injection of said morpholino oligomer at 40µM did not result in a reduction/loss of hemoglobin staining, as expected. However at both 100µM and 200µM concentrations of said morpholino oligomer, staining of hemoglobin appears to be reduced, unlike the complete loss of hemoglobin staining found after 2dpf. Wild-type (wt) embryos showed no signs of changes in hemoglobin staining, indicating that the loss of hemoglobin staining in embryos injected with said morpholino oligomer is specific to the gene 'knock-down' of zfANGPTL1 (Figure 11). Embryos injected with a morpholino oligomer designed specifically against UROD revealed expected results (data not shown).

### Example 3. Whole mount in situ hybridization analysis

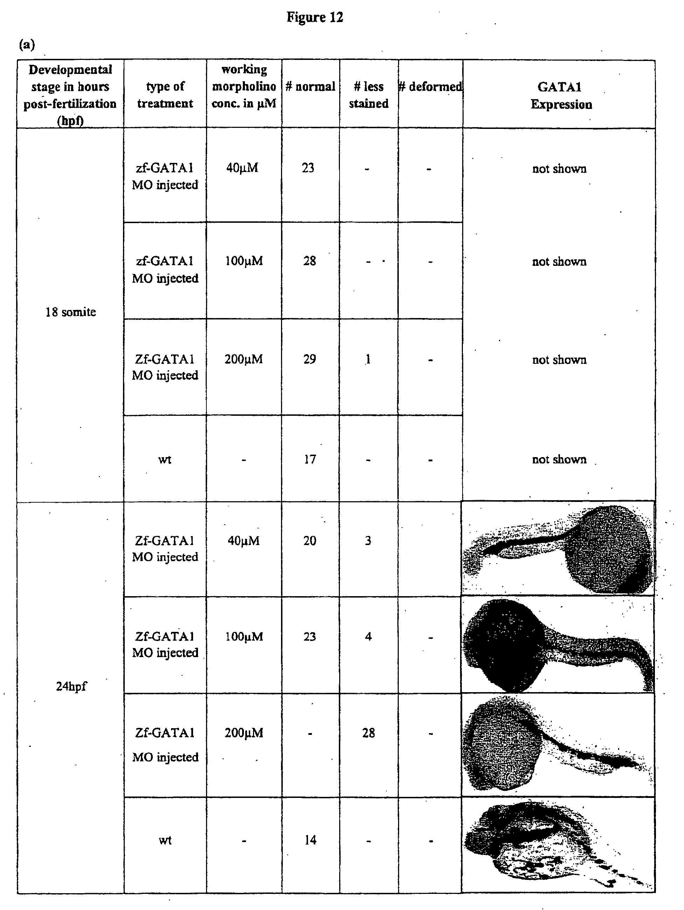

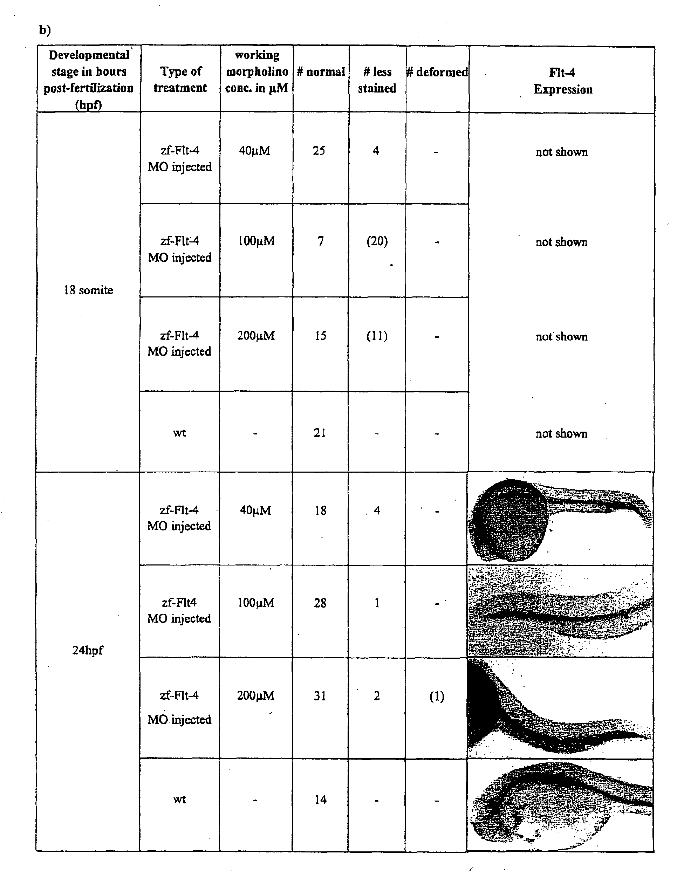

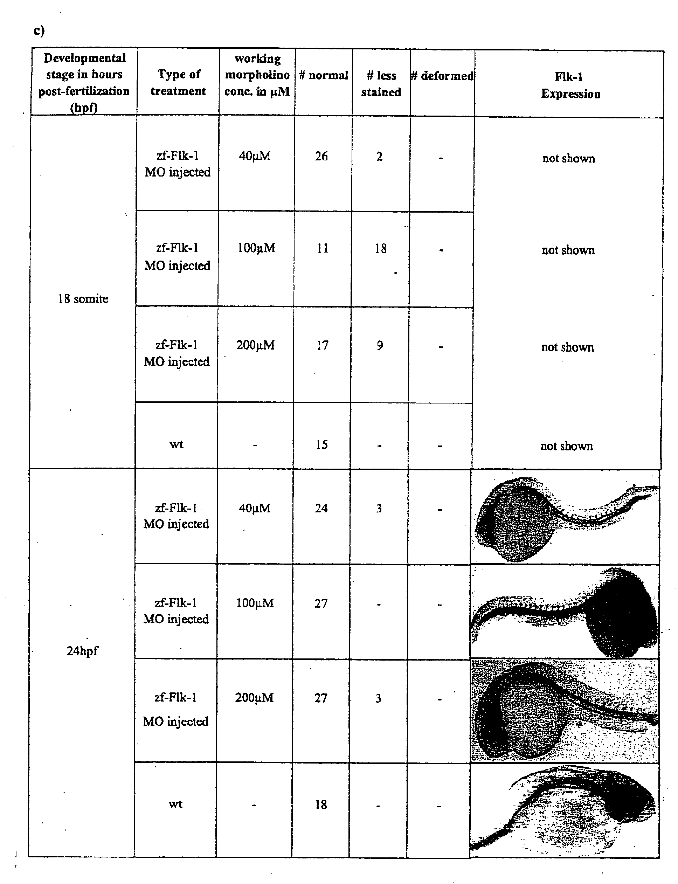

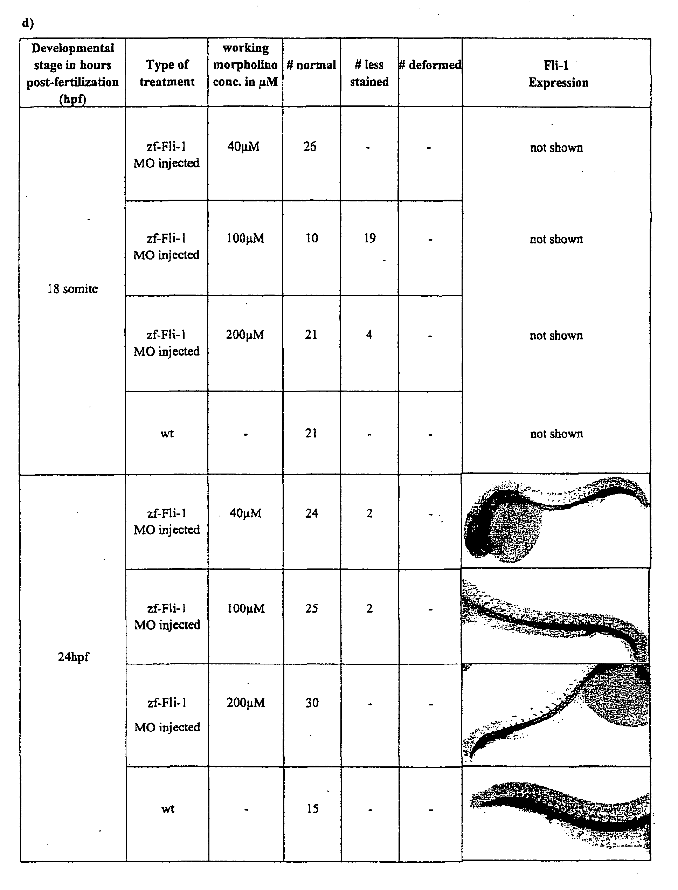

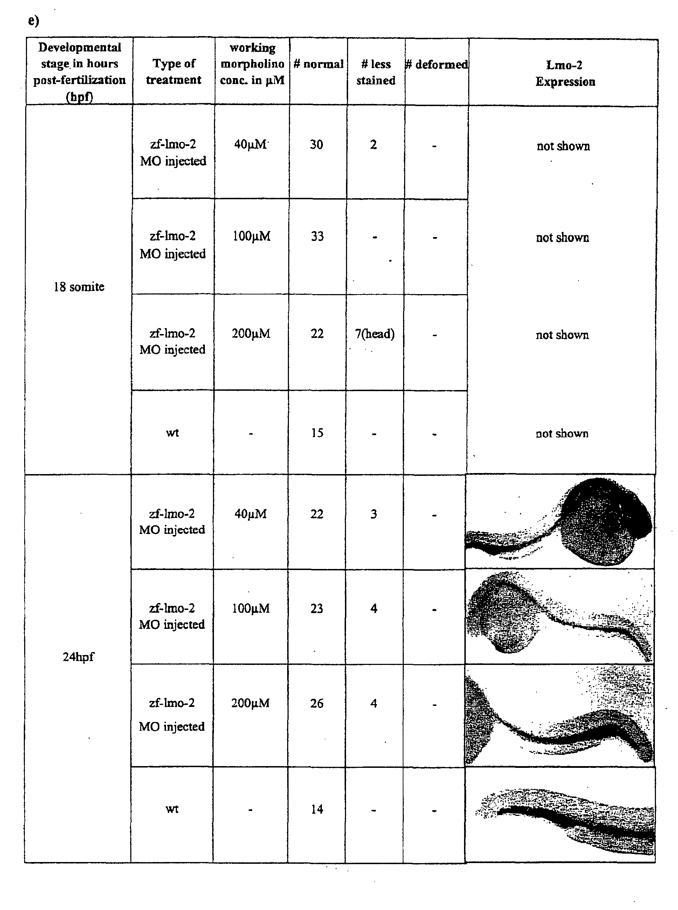

In order to determine whether loss of hemoglobin staining in the initial studies is the direct result of an alteration in hematopoiesis, erythropoiesis and/or angiogenesis due to the ANGPTL1 gene knock-down, wholemount *in situ* hybridisation analysis (Hauptmann, 1999) was performed on treated embryos using tissue-specific genetic markers for blood progenitors and early vasculature (Thompson et al., 1998; Brown et al., 2000; Amatruda and Zon, 1999; Long et al., 1997). Analysis of treated embryos at 18 somite stage and 1dpf for zebrafish GATA-1 marker expression for early erythroid progenitors revealed that ANGPTL1 may not play a role in early hematopoiesis, since early erythroid progenitors are not directly affected by ANGPTL1 gene 'knock-down' (Figur 2a). Furthermore, expression analysis of angiogenesis markers flk-1, fli-1 and flk4, revealed some defects in expression patterns of treated embryos. flk-1 and fli-1 marker (Figure 12c and d) expression of treated embryos is slightly reduced at a morpholino concentration of 100µM, whereas at 40µM and 200µM there is no significant reduction in expression of these two markers. flt-4 marker expression showed a dose-dependent effect in treated embryos at the 18-somite stage; at 40mM the expression pattern of both markers was reduced in a small number of embryos, whereas at 100µM and 200µM the number of embryos with reduced expression increased (Figure 12b), indicating that ANGPTL1 plays a role in angiogenesis. Interestingly, at 24 hpf there is no significant reduction of Flt-4 marker expression in treated embryos. Furthermore, the expression pattern of lmo-1, a marker for primitive hematopoiesis, is unaffected by the 'knock-down' of ANGPTL1 (Figure 12e). that the above results suggest that the loss of hemoglobin staining observed in ANGPTL1 'knockdown' zebrafish embryos clearly demonstrates the role of ANGPTL1 in later, rather than early, stages of erythropoiesis.

SEQUENCE LISTING

<110> Mermaid Pharmaceuticals GmbH

<120> Materials and Methods of Using Angiopoietin-like 1 and
      Angiopoietin-like 2 Genes for Altering Clinical Status

<130> 260-34 EP

<150> US 60/335,362
<151> 2001-10-31

<160> 11

<170> PatentIn Ver. 2.1

<210> 1
<211> 444
<212> DNA
<213> Danio rerio

<400> 1
tgctatgtgt cctcaggtct ggatgttctg ctggttcctc tgtgtgctgt ggtgtgtgcg 60
gatcagcagt tcctccagcc ggcagcgcag gcagatggag gctccagacc cgcagcggca 120
gcagcagtgc tcctacacct tcttaatccc ggagcagaag atcacggggc ctatctgtgc 180
cagccggggg ccctcgctgc cggaccccga gcgcgtgacg cggacggaca tgtctgagct 240
gcggcagacc ctgagccggc agcggcggga gatggagacg ctgcggatga tggtggatgt 300
ggacggagac atggtgcagg agatgaagct gctgcgtaag gagtcgcgga acatgaactc 360
gcgggtcact cagctctaca tgcagctgct gcacgaggtc ctgcgcaagc gcgactcctc 420
tctggagctg gcggcgctgc agaa 444

<210> 2
<211> 2066
<212> DNA
<213> Homo sapiens

<400> 2
atttctccat gtggcagaca gagcaaagcc acaacgcttt ctctgctgga ttaaagacgg 60
cccacagacc agaacttcca ctatactact taaaattaca taggtggctt gtcaaattca 120
attgattagt attgtaaaag gaaaaagaag ttccttctta cagcttggat tcaacggtcc 180
aaaacaaaaa tgcagctgcc attaaagtca cagatgaaca aacttctaca ctgattttta 240
aaatcaagaa taagggcagc aagtttctgg attcactgaa tcaacagaca caaaaagctg 300
gcaatatagc aactatgaag agaaaagcta ctaataaaat taacccaacg catagaagac 360
ttttttttct cttctaaaaa caactaagta aagacttaaa tttaaacaca tcattttaca 420
acctcatttc aaaatgaaga cttttacctg gaccctaggt gtgctattct tcctactagt 480
ggacactgga cattgcagag gtggacaatt caaaattaaa aaaataaacc agagaagata 540
ccctcgtgcc acagatggta aagaggaagc aaagaaatgt gcatacacat tcctggtacc 600
tgaacaaaga ataacagggc caatctgtgt caacaccaag gggcaagatg caagtaccat 660
taaagacatg atcaccagga tggaccttga aaacctgaag gatgtgctct ccaggcagaa 720
gcgggagata gatgttctgc aactggtggt ggatgtagat ggaaacattg tgaatgaggt 780
aaagctgctg agaaaggaaa gccgtaacat gaactctcgt gttactcaac tctatatgca 840
attattacat gagattatcc gtaagaggga taattcactt gaactttccc aactggaaaa 900
caaaatcctc aatgtcacca cagaaatgtt gaagatggca acaagataca gggaactaga 960
ggtgaaatac gcttccttga ctgatcttgt caataaccaa tctgtgatga tcactttgtt 1020
ggaagaacag tgcttgagga tattttcccg acaagacacc catgtgtctc ccccacttgt 1080
ccaggtggtg ccacaacata ttcctaacag ccaacagtat actcctggtc tgctgggagg 1140
taacgagatt cagagggatc caggttatcc cagagattta atgccaccac ctgatctggc 1200
aacttctccc accaaaagcc ctttcaagat accaccggta actttcatca atgaaggacc 1260
attcaaagac tgtcagcaag caaaagaagc tgggcattcg gtcagtggga tttatatgat 1320
taaacctgaa aacagcaatg gaccaatgca gttatggtgt gaaaacagtt tggaccctgg 1380

gggttggact gttattcaga aaagaacaga cggctctgtc aacttcttca gaaattggga 1440
aaattataag aaagggtttg gaaacattga cggagaatac tggcttggac tggaaaatat 1500
ctatatgctt agcaatcaag ataattacaa gttattgatt gaattagaag actggagtga 1560
taaaaaagtc tatgcagaat acagcagctt tcgtctggaa cctgaaagtg aattctatag 1620
actgcgcctg ggaacttacc agggaaatgc aggggattct atgatgtggc ataatggtaa 1680
acaattcacc acactggaca gagataaaga tatgtatgca ggaaactgcg cccactttca 1740
taaaggaggc tggtggtaca atgcctgtgc acattctaac ctaaatggag tatggtacag 1800
aggaggccat tacagaagca agcaccaaga tggaattttc tgggccgaat acagaggcgg 1860
gtcatactcc ttaagagcag ttcagatgat gatcaagcct attgactgaa gagagacact 1920
cgccaattta aatgacacag aactttgtac ttttcagctc ttaaaaatgt aaatgttaca 1980
tgtatattac ttggcacaat ttatttctac acagaaagtt tttaaaatga attttaccgt 2040
aactataaaa gggaacctat aaatgt 2066

<210> 3
<211> 1518
<212> DNA
<213> Homo sapiens

<400> 3
aaccaccatt ttgcaaggac catgaggcca ctgtgcgtga catgctggtg gctcggactg 60
ctggctgcca tgggagctgt tgcaggccag gaggacggtt ttgagggcac tgaggagggc 120
tcgccaagag agttcattta cctaaacagg tacaagcggg cgggcgagtc ccaggacaag 180
tgcacctaca ccttcattgt gccccagcag cgggtcacgg gtgccatctg cgtcaactcc 240
aaggagcctg aggtgcttct ggagaaccga gtgcataagc aggagctaga gctgctcaac 300
aatgagctgc tcaagcagaa gcggcagatc gagacactgc agcagctggt ggaggtggac 360
ggcggcattg tgagcgaggt gaagctgctg cgcaaggaga gccgcaacat gaactcgcgg 420
gtcacgcagc tctacatgca gctcctgcac gagatcatcc gcaagcggga caacgcgttg 480
gagctctccc agctggagaa caggatcctg aaccagacag ccgacatgct gcagctggcc 540
agcaagtaca aggacctgga gcacaagtac cagcacctgg ccacactggc ccacaaccaa 600
tcagagatca tcgcgcagct tgaggagcac tgccagaggg tgccctcggc caggcccgtc 660
ccccagccac cccccgctgc cccgccccgg gtctaccaac cacccaccta caaccgcatc 720
atcaaccaga tctctaccaa cgagatccag agtgaccaga acctgaaggt gctgccaccc 780
cctctgccca ctatgcccac tctcaccagc ctcccatctt ccaccgacaa gccgtcgggc 840
ccatggagag actgcctgca ggccctggag gatggccacg acaccagctc catctacctg 900
gtgaagccgg agaacaccaa ccgcctcatg caggtgtggt gcgaccagag acacgacccc 960
gggggctgga ccgtcatcca gagacgcctg gatggctctg ttaacttctt caggaactgg 1020
gagacgtaca agcaagggtt tgggaacatt gatggcgaat actggctggg cctggagaac 1080
atttactggc tgacgaacca aggcaactac aaactcctgg tgaccatgga ggactggtcc 1140
ggccgcaaag tctttgcaga atacgccagt ttccgcctgg aacctgagag cgagtattat 1200
aagctgcggc tggggcgcta ccatggcaat gcgggtgact cctttacatg gcacaacggc 1260
aagcagttca ccaccctgga cagagatcat gatgtctaca caggaaactg tgcccactac 1320
cagaagggag gctggtggta taacgcctgt gcccactcca acctcaacgg ggtctggtac 1380
cgcgggggcc attaccggag ccgctaccag gacggagtct actgggctga gttccgagga 1440
ggctcttact cactcaagaa agtggtgatg atgatccgac cgaaccccaa caccttccac 1500
taagccagct cccctcc 1518

<210> 4
<211> 1518
<212> DNA
<213> Mus musculus

<400> 4
aaccagcaca ctgcaaggac catgaggcca ctgtgtatga cctactggtg gcttggactg 60
ctggccacgg tcggagctgc tacaggccca gaggctgacg ttgagggcac agaggatggt 120
tcacagagag agtacattta cctcaacagg tacaagcggg caggtgagtc ccccgacaag 180
tgcacctaca ctttcattgt gccccagcag cgggtcacag gtgccatttg tgtcaactcc 240
aaggagcctg aggtgcacct ggagaaccgt gtgcacaagc aggagctgga gctgctcaac 300
aatgagctgc ttaagcagaa gcggcagatc gagacgctgc agcagctggt agaggtagac 360
ggaggcatcg tgagcgaggt gaagctgctg cgcaaggaga gccgcaacat gaactcgaga 420

gtcacgcagc tgtacatgca acttctacat gagatcattc gaaagcgaga caatgcgctg 480
gagctctccc agctggagaa caggatcctg aaccagacag ctgacatgct gcagctggct 540
agcaagtaca aggacctgga gcacaagttc cagcacctgg ctatgctggc acacaaccaa 600
tcagaggtca ttgctcagct cgaagagcac tgccaacgcg tacctgcagc caggcctatg 660
ccccagccac ccccagcagc tccacctcgg gtctaccaac cacccaccta caaccgcatc 720
atcaaccaga tttccaccaa tgagatccag agtgaccaga atctgaaggt gctgccgccc 780
tccttgccca ccatgcctgc ccttaccagt ctcccatctt ccactgataa gccatcaggt 840
ccatggagag actgcctgca agccctggaa gatggtcaca gcaccagctc catctacctg 900
gtgaagcctg agaataccaa ccgcctgatg caggtgtggt gtgaccagag acatgaccct 960
ggaggttgga ctgtcatcca gagacgcctg gatggctctg tcaacttctt caggaactgg 1020
gagacctata agcaagggtt tgggaacatc gatggtgagt actggctggg cctggagaac 1080
atctactggc tgacgaacca aggcaactac aaactgctgg taaccatgga ggactggtct 1140
ggccgtaaag tctttgctga gtatgccagt ttccgactgg agccagaaag cgagtactat 1200
aagctgcggc tggggcgtta tcatggcaat gcaggcgact cctttacctg gcacaacggc 1260
aaacagttta ccaccctgga caggaccatt gatgtctaca caggaaactg tgcccactat 1320
cagaagggag gatggtggta taacgcctgt gctcactcca acctcaatgg ggtctggtac 1380
cgtgggggcc attaccggag cagataccag gacggggtct actgggctga gttccgagga 1440
ggctcttact cactcaagaa ggtggtgatg atgattcggc ccaaccccga tatcttccac 1500
taagctctcc ctgcctgg 1518

<210> 5
<211> 25
<212> DNA
<213> Artificial Sequence

<220>
<223> Description of Artificial Sequence: Antisense
      nucleotide sequence against zebrafish
      angiopoietin-like 1 precursor

<400> 5
acatccagac ctgaggacac atagc 25

<210> 6
<211> 25
<212> DNA
<213> Artificial Sequence

<220>
<223> Description of Artificial Sequence: Antisense
      nucleotide sequence against zebrafish angiopoietin

<400> 6
atggctttct gggatgcgac tcagg 25

<210> 7
<211> 491
<212> PRT
<213> Homo sapiens

<400> 7
Met Lys Thr Phe Thr Trp Thr Leu Gly Val Leu Phe Phe Leu Leu Val
  1               5                  10                  15

Asp Thr Gly His Cys Arg Gly Gly Gln Phe Lys Ile Lys Lys Ile Asn
             20                  25                  30

Gln Arg Arg Tyr Pro Arg Ala Thr Asp Gly Lys Glu Glu Ala Lys Lys

        35                  40                  45

Cys Ala Tyr Thr Phe Leu Val Pro Glu Gln Arg Ile Thr Gly Pro Ile
    50                  55                  60

Cys Val Asn Thr Lys Gly Gln Asp Ala Ser Thr Ile Lys Asp Met Ile
65                  70                  75                  80

Thr Arg Met Asp Leu Glu Asn Leu Lys Asp Val Leu Ser Arg Gln Lys
                85                  90                  95

Arg Glu Ile Asp Val Leu Gln Leu Val Val Asp Val Asp Gly Asn Ile
            100                 105                 110

Val Asn Glu Val Lys Leu Leu Arg Lys Glu Ser Arg Asn Met Asn Ser
        115                 120                 125

Arg Val Thr Gln Leu Tyr Met Gln Leu Leu His Glu Ile Ile Arg Lys
    130                 135                 140

Arg Asp Asn Ser Leu Glu Leu Ser Gln Leu Glu Asn Lys Ile Leu Asn
145                 150                 155                 160

Val Thr Thr Glu Met Leu Lys Met Ala Thr Arg Tyr Arg Glu Leu Glu
                165                 170                 175

Val Lys Tyr Ala Ser Leu Thr Asp Leu Val Asn Asn Gln Ser Val Met
            180                 185                 190

Ile Thr Leu Leu Glu Glu Gln Cys Leu Arg Ile Phe Ser Arg Gln Asp
        195                 200                 205

Thr His Val Ser Pro Pro Leu Val Gln Val Val Pro Gln His Ile Pro
    210                 215                 220

Asn Ser Gln Gln Tyr Thr Pro Gly Leu Leu Gly Gly Asn Glu Ile Gln
225                 230                 235                 240

Arg Asp Pro Gly Tyr Pro Arg Asp Leu Met Pro Pro Pro Asp Leu Ala
                245                 250                 255

Thr Ser Pro Thr Lys Ser Pro Phe Lys Ile Pro Pro Val Thr Phe Ile
            260                 265                 270

Asn Glu Gly Pro Phe Lys Asp Cys Gln Gln Ala Lys Glu Ala Gly His
        275                 280                 285

Ser Val Ser Gly Ile Tyr Met Ile Lys Pro Glu Asn Ser Asn Gly Pro
    290                 295                 300

Met Gln Leu Trp Cys Glu Asn Ser Leu Asp Pro Gly Gly Trp Thr Val
305                 310                 315                 320

Ile Gln Lys Arg Thr Asp Gly Ser Val Asn Phe Phe Arg Asn Trp Glu
                325                 330                 335

Asn Tyr Lys Lys Gly Phe Gly Asn Ile Asp Gly Glu Tyr Trp Leu Gly
            340                 345                 350

Leu Glu Asn Ile Tyr Met Leu Ser Asn Gln Asp Asn Tyr Lys Leu Leu
        355                 360                 365

Ile Glu Leu Glu Asp Trp Ser Asp Lys Lys Val Tyr Ala Glu Tyr Ser
    370                 375                 380

Ser Phe Arg Leu Glu Pro Glu Ser Glu Phe Tyr Arg Leu Arg Leu Gly
385                 390                 395                 400

Thr Tyr Gln Gly Asn Ala Gly Asp Ser Met Met Trp His Asn Gly Lys
                405                 410                 415

Gln Phe Thr Thr Leu Asp Arg Asp Lys Asp Met Tyr Ala Gly Asn Cys
            420                 425                 430

Ala His Phe His Lys Gly Gly Trp Trp Tyr Asn Ala Cys Ala His Ser
        435                 440                 445

Asn Leu Asn Gly Val Trp Tyr Arg Gly Gly His Tyr Arg Ser Lys His
    450                 455                 460

Gln Asp Gly Ile Phe Trp Ala Glu Tyr Arg Gly Gly Ser Tyr Ser Leu
465                 470                 475                 480

Arg Ala Val Gln Met Met Ile Lys Pro Ile Asp
                485                 490

<210> 8
<211> 493
<212> PRT
<213> Homo sapiens

<400> 8
Met Arg Pro Leu Cys Val Thr Cys Trp Trp Leu Gly Leu Leu Ala Ala
  1               5                  10                  15

Met Gly Ala Val Ala Gly Gln Glu Asp Gly Phe Glu Gly Thr Glu Glu
             20                  25                  30

Gly Ser Pro Arg Glu Phe Ile Tyr Leu Asn Arg Tyr Lys Arg Ala Gly
         35                  40                  45

Glu Ser Gln Asp Lys Cys Thr Tyr Thr Phe Ile Val Pro Gln Gln Arg
     50                  55                  60

Val Thr Gly Ala Ile Cys Val Asn Ser Lys Glu Pro Glu Val Leu Leu
 65                  70                  75                  80

Glu Asn Arg Val His Lys Gln Glu Leu Glu Leu Leu Asn Asn Glu Leu
                 85                  90                  95

Leu Lys Gln Lys Arg Gln Ile Glu Thr Leu Gln Gln Leu Val Glu Val
            100                 105                 110

Asp Gly Gly Ile Val Ser Glu Val Lys Leu Leu Arg Lys Glu Ser Arg
        115                 120                 125

Asn Met Asn Ser Arg Val Thr Gln Leu Tyr Met Gln Leu Leu His Glu
    130                 135                 140

Ile Ile Arg Lys Arg Asp Asn Ala Leu Glu Leu Ser Gln Leu Glu Asn
145                 150                 155                 160

Arg Ile Leu Asn Gln Thr Ala Asp Met Leu Gln Leu Ala Ser Lys Tyr
                165                 170                 175

Lys Asp Leu Glu His Lys Tyr Gln His Leu Ala Thr Leu Ala His Asn
            180                 185                 190

Gln Ser Glu Ile Ile Ala Gln Leu Glu Glu His Cys Gln Arg Val Pro
        195                 200                 205

Ser Ala Arg Pro Val Pro Gln Pro Pro Pro Ala Ala Pro Pro Arg Val
    210                 215                 220

Tyr Gln Pro Pro Thr Tyr Asn Arg Ile Ile Asn Gln Ile Ser Thr Asn
225                 230                 235                 240

Glu Ile Gln Ser Asp Gln Asn Leu Lys Val Leu Pro Pro Pro Leu Pro
                245                 250                 255

Thr Met Pro Thr Leu Thr Ser Leu Pro Ser Ser Thr Asp Lys Pro Ser
            260                 265                 270

Gly Pro Trp Arg Asp Cys Leu Gln Ala Leu Glu Asp Gly His Asp Thr
        275                 280                 285

Ser Ser Ile Tyr Leu Val Lys Pro Glu Asn Thr Asn Arg Leu Met Gln
    290                 295                 300

Val Trp Cys Asp Gln Arg His Asp Pro Gly Gly Trp Thr Val Ile Gln
305                 310                 315                 320

Arg Arg Leu Asp Gly Ser Val Asn Phe Phe Arg Asn Trp Glu Thr Tyr
                325                 330                 335

Lys Gln Gly Phe Gly Asn Ile Asp Gly Glu Tyr Trp Leu Gly Leu Glu
            340                 345                 350

Asn Ile Tyr Trp Leu Thr Asn Gln Gly Asn Tyr Lys Leu Leu Val Thr
        355                 360                 365

Met Glu Asp Trp Ser Gly Arg Lys Val Phe Ala Glu Tyr Ala Ser Phe
    370                 375                 380

Arg Leu Glu Pro Glu Ser Glu Tyr Tyr Lys Leu Arg Leu Gly Arg Tyr
385                 390                 395                 400

His Gly Asn Ala Gly Asp Ser Phe Thr Trp His Asn Gly Lys Gln Phe
                405                 410                 415

Thr Thr Leu Asp Arg Asp His Asp Val Tyr Thr Gly Asn Cys Ala His
            420                 425                 430

Tyr Gln Lys Gly Gly Trp Trp Tyr Asn Ala Cys Ala His Ser Asn Leu
        435                 440                 445

Asn Gly Val Trp Tyr Arg Gly Gly His Tyr Arg Ser Arg Tyr Gln Asp
    450                 455                 460

Gly Val Tyr Trp Ala Glu Phe Arg Gly Gly Ser Tyr Ser Leu Lys Lys
465                 470                 475                 480

Val Val Met Met Ile Arg Pro Asn Pro Asn Thr Phe His
                485                 490


<210> 9
<211> 8
<212> PRT
<213> Artificial Sequence

<220>
<221> VARIANT
<222> (1)
<223> Xaa is Asp or Asn

<220>
<221> VARIANT
<222> (2)
<223> Xaa is Lys or His

<220>
<221> VARIANT
<222> (4)
<223> Xaa is Val or Met

<220>
<221> VARIANT
<222> (6)
<223> Xaa is Ala or Thr

<220>
<221> VARIANT
<222> (7)
<223> Xaa is Gly or Arg

<220>
<223> Description of Artificial Sequence: Cabinding
      motif

<400> 9
Xaa Xaa Asp Xaa Tyr Xaa Xaa Asn
  1               5


<210> 10
<211> 12
<212> PRT
<213> Artificial Sequence

<220>
<221> VARIANT
<222> (1)
<223> Xaa is Trp or Cys

<220>
<221> VARIANT
<222> (2)
<223> Xaa is Trp or Glu

<220>
<221> VARIANT
<222> (3)

<223> Xaa is Tyr or Leu

<220>
<223> Description of Artificial Sequence: Fibrinogen
      beta and gamma chains C-terminal domain

<400> 10
Xaa Xaa Xaa Asn Ala Cys Ala His Ser Asn Leu Asn
  1               5                  10


<210> 11
<211> 21
<212> PRT
<213> Homo sapiens

<400> 11
Met Lys Thr Phe Thr Trp Thr Leu Gly Val Leu Phe Phe Leu Leu Val
  1               5                  10                  15

Asp Thr Gly His Cys
             20

### References

Altschul, S.F., Madden, T.L., Schäffer; A.A, Zhang, J., Zhang, Z., Miller, W., and Lipman, D.J. (1997). Gapped BLAST and PSI-BLAST: a new generation of protein database search Programs. Nucleic Acids Res. 25, 3389-3402.

Amatruda, J.F., and Zon, L.I. (1999). Dissecting hematopoiesis and disease using the zebrafish. Dev. Biol. 216, 1-15.

Bemis, L.T., Geske, F.J., and Strange, R. (1995). Use of the yeast two-hybrid system for identifying the cascade of protein interactions resulting in apoptotic cell death. Methods Cell Biol. 46, 139-51

Benton, W.D. and Davis, R.W. (1977). Screening λgt recombinant clones by hybridization to single plaques in situ. Science *196,* 180-182

Bindseil, K.U., Jakupovic, J., Wolf, D., Lavayre, J., Leboul, J., and van der Pyl, D. (2001). Pure compound libraries; a new perspective for natural product based drug discovery. Drug Discov. Today 6, 840-847.

Bradley, A., Evans, M., Kaufman, M.H., Robertson, E. (1984). Formation of germ-line chimaeras from embryo-derived teratocarcinoma cell lines. Nature *309,* 255-256.

Breier, G., Clauss, M., and Risau, W. (1995). Coordinate expression of vascular endothelial growth factor receptor-1 (flt-1) and its ligand suggests a paracrine regulation of murine vascular development. Dev. Dyn. *204*, 228-239.

Brinster, R.L., Chen, H.Y., Trumbauer, M.E., Yagle, M.K., Palmiter, R.D. (1985). Factors affecting the efficiency of introducing foreign DNA into mice by microinjecting eggs. Proc. Natl. Acad. Sci. USA 82, 4438-4442.

Brown, L.A., Rodaway, A.R., Schilling, T.F., Jowett, T., Ingham, P.W., Patient, R.K., and Sharrocks, A.D. (2000). Insights into early vasculogenesis revealed by expression of the ETS-domain transcription factor Fli-1 in wild type and mutant zebrafish embryos. Mech. Dev. *90*, 237-252.

Claesson-Welsh, L. (ed.) (1999). Current Topics in Microbiology and Immunology. (Berlin: Springer Verlag, GmbH & Co), 237, 1-187.

Conklin, D., Gilbertson, D., Taft, D.W., Maurer, M.F., Whitmore, T.E., Smith, D.L., Walker, K.M., Chen, L.H., Wattler, S., Nehls, M., and Lewis, K.B. (1999). Identification of a mammalian angiopoietin-related protein expressed specifically in liver. Genomics *62*, 477-482.

Cull M. G., Miller, J.F., and Schatz, P.J. (1992). Screening for receptor ligands using large libraries of peptides linked to the C terminus of the *lac* repressor. Proc. Natl. Acad. Sci. USA *89,* 1865-1869.

Cwirla, S.E., Peters, E. A., Barrett, R.W., and Dower, W.J. (1990). Peptides on phage: a vast library of peptides for identifying ligands. Proc. Natl. Acad. Sci. USA 87, 6378-6382.

Davis, S., Aldrich, T:H., Jones, P:F., Acheson, A., Compton, D:L., Jain, V., Ryan, T:E., Bruno, J., Radziewski, C., Maisonpierre, P.C., and Yancopoulos, G.D. (1996). Isolation of angiopoietin-1, a ligand for the TIE2 receptor, by secretion-trap expression cloning. Cell. *87,* 1161-1169.

Detrich III, H.W., Kieran, M.W., Chan, F.Y., Barone, L.M., Yee, K., Rundstadler, J.A., Pratt, S., Ransom, D., and Zon, L.I. (1995). Intraembryonic hematopoietic cell migration during vertebrate development. Proc. Natl. Acad. Sci. USA 92, 10713-10717.

Devlin, J.J., Panganiban, L.C., and Devlin, P.E. (1990) Random peptide libraries: a source of specific protein binding molecules. Science *249*, 404-406.

de Vries, C., Escobedo, J.A., Ueno, H., Houck, K., Ferrara, N., and William, L.T. (1992). The fins-like tyrosine kinase, a receptor for vascular endothelial growth factor. Science *255,* 989-991.

DeWitt, S. H., Kiely, J.S., Stankovic, C.J., Schroeder, M.C., Reynolds Cody, D.M., and Pavia, M.R. (1993). "Diversomers": An approach to nonpeptide, nonoligomeric chemical diversity. Proc. Natl. Acad. Sci. USA 90, 6909-6913.

Doetschman, T.C., Eistetter, H., Katz, M., Schmidt, W., and Kemler, R. (1985). The in vitro development of blastocyst-derived embryonic stem cell lines: formation of visceral yolk sac, blood islands and myocardium. J. Embryol. Exp. Morphol. *87*, 27-45.

Ekker, S.C. (2000). Morphants: a new systematic vertebrate functional genomics approach. Yeast *17*, 302-306.

Endo, T., Kitajima, Y., Nishikawa, A., Manase K., Shibuya, M., and Kudo, R. (2001). Cyclic changes in espression of mRNA of vascular endothelial growth factor, its receptors Flt-1 and KDR/Flk-1, and Ets-1 in human corpora lutea. Fertil. Steril. *76*, 762-768.

Erb, E., Janda, K.D., and Brenner, S. (1994). Recursive deconvolution of combinatorial chemical libraries. Proc. Natl. Acad. Sci. USA *91*, 11422-11426.

Evans, M.J., and Kaufman, M.H. (1981). Establishment in culture of pluripotential cells from mouse embryos. Nature *292*, 154-156.

Felici, F., Castagnoli, L., Musacchio, A., Jappelli, R., and Cesareni, G. (1991). Selection of antibody ligands from a large library of oligopeptides expressed on a multivalent exposition vector. J. Mol. Biol. *222*, 301-310.

Fields, S., and Sternglanz, R. (1994). The two-hybrid system: an assay for protein-protein interactions. Trends Genet. 10, 286-292.

Fodor, S.P., Rava, R.P., Huang, X.C., Pease, A.C., Holmes, C.P., and Adams, C.L. (1993). Multiplexed biochemical assays with biological chips. Nature 364, 555-556.

Fong, G-H., Rossant, J., Gertsenstein, M., and Breitman, M.L. (1995). Role of the flt-1 receptor tyrosine kinase in regulating the assembly of vascular endothelium. Nature *376*, 66-70.

Froehler, B., Ng, P., and Matteucci, M. (1988). Phosphoramidate analogues of DNA: synthesis and thermal stability of heteroduplexes. Nucleic Acids Res. *16,* 4831-4839.

Galland, F., Karamysheva, A., Pebusque, M.J., Borg, J.P., Rottapel, R., Dubreuil P., Rosnet, O., and Bimbaum, D. (1993). The FLT4 gene encodes a transmembrane tyrosine kinase related to the vascular endothelial growth factor receptor. Oncogene *8*, 1233-1240.

Gallop, M.A., Barrett, R.W., Dower, W.J., Fodor, S.P., and Gordon, E.M. (1994). Applications of combinatorial technologies to drug discovery. 1. Background and peptide combinatorial libraries. J. Med. Chem. *37*: 1233-1251.

Gordon, E.M., Barrett, R.W., Dower, W.J., Fodor, S.P., and Gallop, M.A. (1994). Applications of combinatorial technologies to drug discovery. II. Combinatorial organic synthesis, library screening strategies and future directions. J. Med. Chem. 37, 1385-1401.

Gossler, A., Doetschman, T., Kom, R., Serfling, E., Kemler, R (1986). Transgenesis by means of blastocyst-derived embryonic stem cell lines. Proc. Natl. Acad. Sci. USA *83,* 9065-9069.

Grabley, S., Thiericke, R., and Sattler, I. (2000). Tools for drug discovery: natural product-based libraries.
Ernst Schering Res. Found. Workshop. 32, 217-252.

Hamaguchi, I., Huang, X-L., Takakura, N., Tada, J-I., Yamaguchi, Y., Hiroaki, K., and Suda, T. (1999). In vitro hematopoietic and endothelial cell development from cells expressing TEK receptor in murine aorta-gonad-mesonephros region. Blood 93, 1549-1556.

Hattori, K., Dias, S., Heissig, B., Hackett, N.R., Lynden, D., Tateno, M., Hicklin, D.J., Zhu, Z., Witte, L., Crystal, R.G., Moore, M.A., and Rafii, S. (2001). Vascular endothelial growth factor and angiopoietin-1 stimulate postnatal hematopoiesis by recruitment of vasculogenic and hematopoietic stem cells. J. Exp. Med. 193, 1005-1014.

Hauptmann, G. (1999). Two-color detection of mRNA transcript localizations in fish and fly embryos using alkaline phosphatase and β-galactosidase conjugated antibodies. Dev. Genes. Evol. *209*, 317-321.

Houghten, RA., Wilson, D.B., and Pinilla, C. (2000). Drug discovery and vaccine development using mixture-based synthetic combinatorial libraries. Drug Discov. Today *5*, 276-285.

Houghten, R.A., Appel, J.R., Blondelle, S.E., Cuervo, J.H., Dooley, C.T, and Piunilla, C. (1992). The use of synthetic peptide combinatorial libraries for the identification of bioactive peptides. Biotechniques. *13*, 412-421.

Huang, H., Vogel, S.S., Liu, N., Melton, D.A., and Lin, S. (2001). Analysis of pancreatic development in living transgenic zebrafish embryos. Mol. Cell. Endocrinol. *177*, 117-124.

Huang, X.L., Takakura, N., and Suda, T. (1999). In vitro effects-of angiopoietins and VEGF on hematopoietic and endothelial cells. Biochem. Biophys. Res. Commun. *264*, 133-138.

Hyrup, B., and Nielsen, P.E. (1996). Peptide nucleic acids (PNA): synthesis, properties and potential applications. Bioorg. Med. Chem. *4*, 5-23.

Jaenisch, R. (1988). Transgenic animals. Science. *240*, 1468-1474.

Jaenisch, R. (1976). Germ line integration and Mendelian transmission of the exogenous Moloney leukemia virus. Proc Natl Acad Sci. USA 73, 1260-1264.

Jahner, D., Stuhlmann, H., Stewart, C.L., Harbers, K., Lohler, J., Simon, I., and Jaenisch, R. (1982). De novo methylation and expression of retroviral genomes during mouse embryogenesis. Nature 298, 623-628.

Jahner, D., Haase, K., Mulligan, R., and Jaenisch R. (1985). Insertion of the bacterial gpt gene into the germ line of mice by retroviral infection. Proc. Natl. Acad. Sci. USA *82*, 6927-31.

Jowett, T. (2001). Double in situ hybridisation techniques. Methods *23*, 345-358.

Kaipainen, A., Korhonen, J., Mustonen, T., van Hinsbergh, V.W., Fang, G.H., Dumont, D., Breitman, M., and Alitalo, K. (1995). Expression of the fins-like tyrosine kinase 4 gene becomes restricted to lymphatic endothelium during development. Proc. Natl. Acad. Sci. USA *92*, 3566-3570.

Khoury, G., and Gruss, P. (1983). Enhancer Elements. Cell *33*, 313-314.

Kim, I., Kwak, H.J., Ahn, J.E., So, J-N., Liu, M., Koh, K.N., and Koh, G.Y. (1999a). Moloecular cloning and charaterizazion of a novel angiopoietin family protein, angiopoietin-3. FEBS Lett. *443*, 353-356.

Kim, I., Moon, S-O., Koh, K.N., Kim, H., Uhm, C-S., Kwak, H.J., Kim, N-G., Koh, G.Y. (1999b). Molecular cloning, expression and charaterization of angiopoietin-relate protein. J. Biol. Chem. 274, 26523-26528.

Kim, I., Kim, H.G., So, J-N., Kim, J.H., Kwak, H.J., and Koh, G.Y. (2000). Angiopoietin-1 regulates endothelial cell survival through the phosphatidylinositol 3'-kinase/Akt signal transduction pathway. Circ. Res. *86*, 24-29.

Kontos, C.D., Stauffer, T.P., Yang, W.P., York, J.D., Huang, L., Blanar, M.A., Meyer, T., and Peters, K.G. (1998). Tyrosine 1101 of Tie2 in the major site of association of p85 and is required for activation of phosphatidylinositol 3-kinase and Akt. Mol. Cell Biol. *18*, 4131-4140.

Koster, R.W., and Fraser, S.E. (2001). Tracing transgene expression in living zebrafish embryos. Dev. Biol. 233, 329-346

Kwak, H.J., So, J-N., Lee, S.J., Kim, I., and Koh, G.Y. (1999). Angiopoietin-1 is an apoptosis survival factor for endothelial cells. FEBS Lett. *448*, 249-253.

Lakso, M., Sauer, B., Mosinger, B. Jr., Lee, E.J., Manning, R.W., Zu, S.H., Mulder, K.L., and Westphal, H. (1992). Targeted oncogene activation by site-specific recombination in transgenic mice. Proc. Nad. Acad. Sci. USA *89*, 6232-6236.

Lam, K.S., Salmon, S.E., Hersh, E.M., Hruby, V.J., Kazmierski, W.M., and Knapp, R.J. (1991). A new type of synthetic peptide library for identifying ligand-binding activity. Nature *354*, 82-84.

Lemaitre, M., Bayard, B., and Lebleu, B. (1987). Specific antiviral activity of a poly (L-lysine)-conjugated oligodeoxyribonucleotide sequence complementary to vesicular stomatitis virus N protein mRNA initiation site. Proc. Natl. Acad. Sci. USA *84*, 648-652.

Letsinger, R.L., Zhang, G.R., Sun, D.K., Ikeuchi, T., and Sarin, P.S. (1989). Cholesteryl-conjugated oligonucleotides: synthesis, properties, and activity as inhibitors of replication of human immunodeficiency virus in cell culture. Proc. Natl. Acad. Sci. USA *86*, 6553-6556.

Liang, D., Chang, J.R, Chin, A.J., Smith, A., Kelly, C., Weinberg, E.S., Ge, R. (2001). The role of vascular endothelial growth factor (VEGF) in vasculogenesis, angiogenesis, and hematopoiesis in zebrafish development. Mech. Dev. *108,* 29-43.

Long, Q., Meng, A., Wang, H., Jessen, J.R., Farrell, M.J., and Lin, S. (1997). GATA-1 expression pattern can be recapitulated in living transgenic zebrafish using GFP reporter gene. Development. *124*, 4105-4111.

Lyons, M.S., Bell, b., Stainier, D., and Peters, K.G. (1998). Isolation of the zebrafish homologues for the tie-1 and tie-2 endothelium-specific receptor tyrosine kinases. Dev. Dyn. *212*, 133-140.

Meng, A., Jessen, J.R. and Lin, S. (1999). Transgenesis. Methods Cell Biol. *60*, 133-148.

Maisonpierre, P.C., Suri, C., Jones, P.F., Bartunkova, S., Wiegand, S.J., Radziejewski, C., Compton, D., McClain, J., Aldrich, T.H., Papadopoulos, N., Daly, T.J., Davis, S., Sato, T.N., and Yancopoulos, G.D. (1997). Angiopoietin-2, a natural antagonist for Tie2 that disrupts in vivo angiogenesis. Science *277,* 55-60.

Millauer, B., Wizigmann-Voos, S., Schnurch, H., Martinez, R., Moller, N.P., Risau, W., and Ullrich, A. (1993). High affinity VEGF binding and developmental expression suggest Flk-1 as a major regulator of vasculogenesis and angiogenesis. Cell 72, 835-46.

Miller. (1989). Oligonucelotides: Antisense Inhibitors of Gene Expression: a formacetal or analog thereof, as well as α-DNA. pp. 82-85.

Minden, A., Lin, A., Smeal, T., Derijard, B., Cobb, M., Davis, R., and Karin, M. (1994). C-Jun N-terminal phosphorylation correlates with activation of the JNK subgroup but not the ERK subgroup of mitogen-activated protein kinases. Mol. Cell. Biol. *14*, 6683-6688.

Motoike, T., Loughna, S., Perens, E., Roman, B.L., Liao, W., Chau, T.C., Richardson, C.D., Kawate, T., Kuno, J., Weinstein, B.M., Stainier, D.Y., and Sato, T.N. (2000). Universal GFP reporter for the study of vascular development. Genesis *28,* 75-81

Mustonen, T., and Alitalo, K. (1995). Endothelial receptor tyrosine kinase involved in angiogenesis. J. Cell Biol. 129, 895-898.

Nasevicius, A., and Ekker, S.C. (2000). Effective targeted gene 'knockdown' in zebrafish. Nat. Genet. 26, 216-220.

Neufeld, G., Cohen, T., Gengrinovitch, S., and Poltoral, Z. (1999). Vascular endothelial growth factor (VEGF) and its receptors. FASEB J. *13*, 9-22.

Nielsen, P.E. (2000). Antisense peptide nucleic acids. Curr. Opin. Mol. Ther. 2, 282-287.

O'Gorman, S., Fox, D.T., and Wahl, G.M. (1991). Recombinase-mediated gene activation and site-specific integration in mammalian cells. Science *251*, 1351-1355.

Orban, P.C., Chui, D., and Marth, J.D. (1992). Tissue- and site-specific DNA recombination in transgenic mice. Proc. Natl. Acad. Sci. USA *89*, 6861-6865.

Orum, H., and Wengel, J. (2001). Locked nucleic acids: a promising molecular family for genefunction analysis and antisense drug development. Curr. Opin. Mol. Ther. *3*, 239-243.

Palmer, A., Gavin, AC., and Nebreda, A.R. (1998). A link between MAP kinase and p34^{cdc2}/cyclin b during oocyte maturation: p90^{rsk} phosphorylates and inactivates the p34^{cdc2} inhibitory kinase Mytl. EMBO J. *17*, 5037-5047.

Pham, A.N., Roman, B.L., and Weinstein, B.M. (2001). Isolation and expression analysis of three zebrafish angiopoietin genes. Dev. Dyn. *221*, 470-474.

Procopio, W.N., Pelavin, P.I., Lee, W.M.F., and Yielding, N.M. (1999). Angiopoietin-1 and -2 coiled coil domains mediate distinct homo-oligomerization patterns, but fibrinogen-like domains mediate ligand activity. J. Biol. Chem. 274, 30196-30201.

Rader, C. (2001). Antibody libraries in drug and target discovery. Drug Discov. Today *6*, 36-43.

Rayner, B., Malvy, C., Paolem, J., Lebleu, B., Paoletti, C., and Imbach, J. (1989). α-oligodeoxynucleotide analogues. In Oligonucelotides: Antisense Inhibitors of Gene Expression. J. Cohen, ed. (Boca Raton, FL: CRC Press, Inc.), pp.119-136.

Risau, W. (1991). Vasculogenesis, angiogenesis and endothelial cell differentiation during embryonic development. In The development of the vascular system, R.N. Feinberg, G.K. Shere, and R. Auerbach, eds. (Basel: Karger), pp. 58-68.

Risau, W. (1997). Mechanisms of angiogenesis. Nature *386,* 671-674.

Robertson, E., Bradley, A., Kuehn, M., and Evans, M. (1986). Germ-line transmission of genes introduced into cultured pluripotential cells by retroviral vector. Nature *323*, 445-448.

Rodriguez, R., and Chamberlin, M. (eds.) (1982). Promoters: Structure and Function. (New York: Praeger).

Saiki, R.K., Gelfand, D.H., Stoffel, S., Scharf, S.J., Higuchi, R., Horn, G.T., Mullis, K.B., and Erlich, H.A. (1988). Primer-directed enzymatic amplification of DNA with a thermostable DNA polymerase. Science *239*, 487-491.

Saragovi, H.U., Greene, M.I., Chrusciel, R.A., and Kahn, M. (1992). Loops and secondary structure mimetics: development and applications in basic science and rational drug design. Biotechnology 10, 773-778.

Sarin, P.S., Agrawal, S., Civeira, M.P., Goodchild, J., Ikeuchi, T., and Zamecnik, P.C. (1988). Inhibition of acquired immunodeficiency syndrome virus by oligodeoxynucleoside methylphosphonates. Proc. Natl. Acad. Sci. USA 85, 7448-7451.

Scott, J.K., and Smith, G.P. (1990). Searching for peptide ligands with an epitope library. Science 249, 386-390.

Schaefer, B. (1995). Revolution in rapid amplification of cDNA ends: new strategies for polymerase chain reaction cloning of full-length cDNA ends. Anal. Biochem. 227, 255-273

Shalaby, F., Rossant, J., Yamaguchi, T.P., Gertenstein, M., Wu, X-F., Breitman, M.L., and Schuh, A.C. (1995). Failure of blood island formation and vasculogenesis in flk-ldeficient mice. Nature 376, 62-66.

Shibahara, S., Mukai, S., Morisawa, H., Nakashima, H., Kobayashi, S., and Yamamoto, N. (1989). Inhibition of human immunodeficiency virus (HTV-1) replication by synthetic oligo-RNA derivatives. Nucleic Acids Res. *17*, 239-252.

Shibuya, M., Yamaguchi, S., Yamane, A., Ikeda, T., Tojo, A., Matsushime, H., and Sato, M. (1990). Nucleotide sequence and expression of a novel human receptor-type tyrosine kinase gene (fit) closely related to the fins family. Oncogene 5, 519-524.

Stein, C., and Cohen, J. (1989). Phosphorothioate oligonucleotide analogs. In Oligonucelotides: Antisense Inhibitors of Gene Expression. J. Cohen, ed. (Boca Raton, FL: CRC Press, Inc.), pp.97-117.

Stein, C.A., Subasinghe, C., Shinozuka, K., and Cohen, J.S. (1988). Physicochemical properties of phosphorothioate oligodeoxynucleotides. Nucleic Acids Res. 16, 3209-3221.

Summerton, J. (1999). Morpholino antisense oligomers: the case for an RNase H-independent structural type. Biochim. Biophys. Acta. *1489,* 141-158.

Summerton, J., and Weller, D. (1997). Morpholino antisense oligomers: design, preparation, and properties. Antisense Nucleic Acid Drug Dev. 7, 187-195.

Takakura, N., Huang, X.L., Naruse, T., Hamaguchi, I., Dumont, D.J., Yancopoulos, G.D., and Suda, T. (1998). Critical role of the TIE2 endothelial cell receptor in the development of definitive hematopoiesis. Immunity 9, 677-686.

Terman, B.I., Dougher-vermazen, M., Carrion, M.E., Dimitrov, D., Armellino, D.C., Gospodarowicz, D., and Bohlen, P. (1992). Identification of the KDR tyrosine kinase as a receptor for vascular endothelial cell growth factor. Biochem. Biophys. Res. Commun. *187*, 1579-1586.

Thompson, M.A., Ransom, D.G., Pratt, S.J., MacLennan, H., Kieran, M.W., Detrich, H.W., Vail, B., Huber, T.L., Paw, B., Brownlie, A.J., Oates, A.C., Fritz, A., Gates, M.A., Amores, A., Bahary, N., Talbot, W.S., Her, H., Beier, D.R, Postlethwait, J.H., and Zon, L.I. (1998). The *cloche* and *spadetail* genes differentially affect hematopoiesis and vasculogenesis. Dev. Biol. *197*, 248-269.

Topcu, Z. and Borden, K.L. (2000). The yeast two-hybrid system and its pharmaceutical significance. Pharm Res. *17*, 1049-55.

Valenzuela, D.m., Griffiths, J.A., Rojas, J., Aldrich, T.H., Jones, P.F., Zhou, H., McClain, J., Copeland, N.G., Gilbert, D.J., Jenkins, N.A., Huang, T., Papadopoulos, N., Maisonpierre, P.C., Davis, S., and Yancopoulos, G.D. (1999). Angiopoietins 3 and 4: diverging gene counterparts in mice and humans. Proc. Natl. Acad. Sci. USA 96, 1904-1090.

van der Krol, A.R., Mol, J.N., and Stuitje, A.R. (1988). Modulation of eukaryotic gene expression by complementary RNA or DNA sequences. Biotechniques 6, 958-976.

van der Putten, H., Botteri, F.M., Miller, A.D., Rosenfeld, M.G., Fan, H., Evans, R.M., and Verma, I.M. (1985). Efficient insertion of genes into the mouse germ line via retroviral vectors. Proc. Natl. Acad. Sci. USA 82, 6148-6152.

Wagner, R. W. (1994). Gene inhibition using antisense oligodeoxynucleotides. Nature *372,* 333-335

Wahlestedt, C., Salmi, P., Good, L., Kela, J., Johnsson, T., Hokfelt, T., Broberger, C., Porreca, F., Lai, J., Ren, K., Ossipoy, M., Koshkin, A., Jakobsen, N., Skouv, J., Oerum, H., Jacobsen, M.H., and Wengel, J. (2000). Potent and nontoxic antisense oligonucleotides containing locked nucleic acids. Proc. Natl. Acad. Sci. USA 97, 5633-5638.

Westerfield, M. (2000). The Zebrafish Book, Institute of Neuroscience, University of Oregon.

Xu, W-F., Chung, D.W., and Davie, W.E. (1996). The assmebly of human fibrinogen. J. Biol. Chem. *271*, 27948-27953.

Zhang, B., Kraemer, B., SenGupta, D., Fields, S. and Wickens, M. (1999). Yeast three-hybrid system o detect and analyse interactions between RNA and protein. Methods Enzymol. *306,* 93-113.

Zon, G. (1988). Oligonucleotide analogues as potential chemotherapeutic agents.
Pharm Res. 5, 539-549.

### ADDITIONAL REFERENCES

The Polymerase Chain Reaction. Current Protocols In Molecular Biology, John Wiley & Sons, N.Y. (1989), Vol-3, 15.0.3-15.8.7.

Preparation of Poly (A)⁺ RNA. Current Protocols In Molecular Biology, John Wiley & Sons, N.Y. (1989), Vol-1, 4.5.1-4.5.3.

| US Patent Documents: | | |
|---|---|---|
| 4,683,195 | Mullis et al. | Jul., 1987 |
| 4,833,080 | Brent et al. | May, 1989 |
| 5,185,444 | Summerton et al. | Feb., 1993 |
| 5,217,866 | Summerton et al. | Jun., 1993 |
| 5,223,409 | Lander et al. | Jun., 1993 |
| 5,403,484 | Lander et al. | April, 1995 |
| 5,434,131 | Lindsley et al. | Jul., 1995 |
| 5,571,698 | Lander et al. | Nov., 1996 |

| Foreign Patent Documents: | | | |
|---|---|---|---|
| 89/10134 | Pardridge et al. | Nov., 1989 | WO |
| 88/09810 | Tullis, R. | Feb., 1990 | WO |
| 92/15694 | Wahl et al. | Sept., 1992 | WO |

### Other References Cited:

Kim et al., J Biol Chem (1999) Accession No. AF125176

Kim et al., J Biol Chem (1999) Accession No. NM_012098

NCBI Annotation Project (2001) Accession No. XM_001720

Sugano et al. unpublished (1999) Accession No. AW174181

## Claims

1. A pharmaceutical composition comprising angiopoietin-like 1 (ANGPTL1) or angiopoietin-like 2 (ANGPTL2) protein, a fragment or a derivative thereof.

2. The composition of claim 1, wherein the composition comprises a polypeptide, which is at least 55% identical with the sequence shown in SEQ ID NO: 7 or SEQ ID NO: 8, a fragment or derivative thereof.

3. A pharmaceutical composition comprising a polynucleotide sequence encoding ANGPTL1 or ANGPTL2, a fragment, or a derivative thereof.

4. The composition of 3, wherein the polynucleotide sequence is at least 70% identical with a polynucleotide sequence which encodes the sequence shown in SEQ ID NO: 7 or SEQ ID NO: 8, a fragment or a derivative thereof.

5. The composition of 3, wherein the polynucleotide sequence is at least 70% identical with the polynucleotide sequence shown in SEQ ID NO: 2 or SEQ ID NO: 3.

6. The composition of any of claims 1-5, further comprising a suitable carrier.

7. The composition of claims 3-5, wherein the polynucleotide sequence is operably linked to an expression control sequence.

8. The composition of claim 7, wherein the polynucleotide sequence operably linked to an expression control sequence is in a host cell.

9. The composition of 8, wherein the host cell is a yeast, insect, teleost, amphibian or mammalian cell.

10. The composition of any of claims 3-9, wherein the polynucleotide sequence is in a delivery complex.

11. The composition of 10, wherein the delivery complex is a sterol, lipid or virus.

12. A method of screening for a therapeutic agent for the treatment of blood-related disorders or a defect in vasculature comprising:
a. contacting ANGPTL1 polypeptide, ANGPTL2 polypeptide, fragment or derivative thereof with a test compound or molecule or a library of test molecules or compounds, under conditions to allow specific binding and/interaction; and
b. detecting the level of specific binding and/interaction,
wherein an alteration in the level of interaction relative to a control indicates a potential therapeutic activity.

13. A method of screening for a therapeutic agent for the treatment of blood-related disorders or a defect in vasculature comprising:
a. contacting a reporter construct under the control of ANGPTL1 or ANGPTL2 promoter with a test molecule or compound or a library of test molecules or compounds, under conditions to allow specific binding and/interaction; and
b. detecting the level of expression of the reporter construct,
wherein an alteration in the level of expression to a control indicates a potential therapeutic activity.

14. The method of claims 12 and 13, wherein the library of test molecules or compounds is selected from the group consisting of DNA molecules, peptides, agonists, antagonists, monoclonal antibodies, immunoglobulins and pharmaceutical agents.

15. A method for treating blood-related disorders or defects in vasculature in a human patient comprising:
a. providing a composition that comprises a therapeutic agent identified in the method of any one of claims 12-14; and
b. administering a therapeutically effective amount of the composition from (a) to the patient.

16. The method of claim 15, wherein the test molecules or compounds are in a suitable carrier.

17. A method for treating blood-related disorders or defects in vasculature, comprising administering to a patient a therapeutically effective amount of a polypeptide comprising ANGPTL1 or ANGPTL2, or a fragment or a derivative thereof.

18. The method of claim 12 or 17, wherein the polypeptide is at least 55% identical with the sequence shown in SEQ ID NO: 7 or SEQ ID NO: 8, a fragment or derivative thereof.

19. A method for treating blood-related disorders or defects in vasculature, comprising administering to a patient a therapeutically effective amount of polynucleotide sequence encoding ANGPTL1 or ANGPTL2, or a fragment or a derivative thereof.

20. The method of claim 19, wherein the polynucleotide sequence is at least 70% identical with the polynucleotide sequence which encodes the sequence shown in SEQ ID NO: 7 or SEQ ID NO: 8, a fragment or a derivative thereof.

21. The method of any one of claims 12-20, wherein the blood-related disorder is leukemia or anemia.

22. A method for modulating proliferation and/or differentiation or cell death of target cells comprising administering a biologically active amount of an ANGPTL1 or ANGPTL2 polypeptide or polynucleotide to said target cells.

23. The method of claim 22, wherein said target cells are hematopoietic stem cells, erythroid or endothelial cells.

24. A method for determining whether a subject is at risk of developing or has a blood related disease or a defect in vasculature caused by angiopoietin-like proteins comprising determining the angiopoietin-like allele status of said subject; an altered angiopoietin-like allele status compared to a control indicating that said subject is at risk of developing or has a blood-related disease or a defect in vasculature caused by angiopoietin-like proteins.

25. A kit identifying a subject at risk of developing a blood-related disease or a defect in vasculature or having a blood-related disease or subject with a defect in vasculature caused by angiopoietin-like proteins, comprising means for measuring the level of an angiopoietin-like protein encoding polynucleotide sequence in a sample of cells of said subject and instructions to use the kit.

26. A transgenic non-human animal which does not produce functional ANGPTL1 and/or ANGPTL2 protein, or which produces sub-optimal levels of ANGPTL1 and/or ANGPTL2 protein, or which overproduces the ANGPTL1 and/or ANGPTL2 protein.

27. The animal of claim 26, wherein the genome of the animal comprises a mutation in the ANGPTL1 or ANGPTL2 genes.

28. The animal of claim 27, wherein said mutation is a homozygous disruption.

29. The animal of claim 27, wherein said mutation is a insertion, deletion, or a substitution mutation.

30. The animal of claim 27, wherein said mutation is generated by gene targeting, gene trap integration, or mutagenesis.

31. The animal of claim 27, wherein the mutation has occurred in an exon, intron, regulatory region or splicing site of the ANGPTL1 and/or ANGPTL2 genes.

32. The animal of any of the claims 26-31, wherein said animal exhibits a blood-related disease or a defect in vasculature.

33. The animal of any of the claims 26-32, wherein said animal is a mammal, amphibian, teleost or insect.

34. An antisense oligomer, wherein said oligomer is complementary to mRNA encoding ANGPTL1 or ANGPTL2, and wherein said antisense oligomer is from 6 to 50 nucleotides in length.

35. The oligomer of claim 34, wherein the antisense oligomer is complementary to the translation initiation site of ANGPTL1 or ANGPTL2.

36. The oligomer of claim 34, wherein said antisense oligomer is complementary to the polynucleotide sequence of SEQ ID NO: 1, SEQ ID NO: 2, SEQ ID NO: 3, or SEQ ID NO: 4.

37. The oligomer of any of claims 34 to 36, wherein the antisense oligomer comprises the nucleotide sequence of SEQ ID: 5 or SEQ ID NO:6.

38. The oligomer of any of claims 34-37, wherein binding of said oligomer to said mRNA is effective in altering transcription or translation of said mRNA in a host cell expressing said mRNA.

39. The oligomer of claim 38, wherein the host cell is a bacterial, yeast, insect, or vertebrate cell.

40. The oligomer of any of claims 34-39, wherein the antisense oligomer is in a delivery complex.

41. The oligomer of claim 40, wherein the delivery complex is a sterol, lipid, virus.

42. The oligomer of claims 34-41, wherein said oligomer comprises:
a. at least one modified base moiety, preferably selected from the group consisting of 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5¹-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-Soxyacetic acid methylester, uracil-5-oxyacetic acid (v), - 5-methyl-2-thiouracil, 3-(3-amino-3-N-2-carboxypropyl) uracil, (acp3)w, and 2,6-diaminopurine, and/or
b. at least one modified sugar moiety, preferably selected from the group consisting of arabinose, 2-fluoroarabinose, xylulose, and hexose, and/or
c. at least one modified phosphate backbone, preferably selected from the group consisting of phosphorothioate, phosphoramidothioate, phosphoramidate, phosphordiamidate, methylphosphonate, alkyl phosphotriester, formacetal or analog thereof, as well as α-DNA, 2'-O-methyl RNA, and morpholine backbone.
